# FASCICULE DE BREVET EUROPEEN

(11) **EP 0 600 762 B1**
(45) Date de publication et mention de la délivrance du brevet: **10.07.1996**
(21) Numéro de dépôt: 93402795.4
(22) Date de dépôt: 17.11.1993
(51) Int. Cl.: C07C 279/12, A61K 31/16, A61K 31/155

(54) **Analogues de la 15-déoxyspergualine, leur procédé de préparation et leur utilisation en thérapeutique en tant qu'agents immunosuppreseurs**
15-Deoxyspergualin analoge Verbindungen, Verfahren zu ihrer Herstellung und ihre Verwendung als Therapeutika zur Unterdrückung der Immunabwehr
Analogues of the 15-deoxyspergualine, process for their preparation and their use as immunosuppresants

(30) Priorité: 02.12.1992 FR 9214517
(43) Date de publication de la demande: 08.06.1994
(73) Titulaire: FOURNIER INDUSTRIE ET SANTE, F-75008 Paris (FR)
(72) Inventeur: Renaut, Patrice, F-21121 Hauteville-lès-Dijon (FR); Lebreton, Luc, F-21000 Dijon (FR); Dutartre, Patrick, F-21110 Longchamp (FR); Derrepas, Philippe, F-21410 Agey (FR); Samreth, Soth, F-21600 Longvic (FR)
(74) Mandataire: Clisci, Serge

(56) Documents cités:
- EP-A- 0 105 193
- EP-A- 0 347 820
- JOURNAL OF ANTIBIOTICS vol. 41, no. 11 , Novembre 1988 , TOKYO JP pages 1629 - 1643 R. NISHIZAWA 'Synthesis and Biological Activity of Spergualin Analogues I'

## Description

La présente invention a trait à de nouveaux composés de structure apparentée à celle de la 15-déoxyspergualine. Elle concerne également leur procédé de préparation et leur utilisation en thérapeutique en tant qu'agents immunosuppresseurs.

### ART ANTERIEUR

On sait que la 15-déoxyspergualine est un dérivé de la spergualine, cette dernière étant un antibiotique isolé d'une culture de Bacillus laterosporus. Les premières études de la 15-déoxyspergualine ont mis en évidence une activité antitumorale et, plus tard, l'étude de son activité dans le domaine de l'immunosuppression est devenue prépondérante.

A ce sujet, on pourra notamment se référer aux publications suivantes: G.DICKNEITE, "15-Deoxyspergualin : From Cytostasis to Immunosuppression", Behring Inst. Mitt., N° 82,231-239 (1988) ; G.DICKNEITE, "The Influence of (+)-15-Deoxyspergualin on Experimental Transplantation and its Immunopharmacological Mode of Action", Behring Inst. Mitt., N° 80,93-102 (1986) et K. NEMOTO, "Deoxyspergualin in lethal murine graft-versus-host disease", Transplantation Vol.51, 712-715, N°3, March 1991.

Malgré une réelle activité dans le domaine de l'immunosuppression, la 15-déoxyspergualine ne présente pas une stabilité chimique satisfaisante. On a donc cherché à obtenir des dérivés plus stables, notamment en remplaçant le résidu α-hydroxyglycine de la déoxyspergualine par différents α ou ω-aminoacides.

A ce sujet, on pourra notamment se référer à R. NISHIZAWA, "Synthesis and biological activity of spergualin analogues", J.Antibiotics 1988, 42 (11), 1629-1643 et à EP-A-0 105 193.

### OBJET DE L'INVENTION

On propose à présent de nouveaux analogues de la 15-déoxyspergualine, qui sont structurellement différents des produits proposés dans EP-A-0 105 193, sont chimiquement stables et présentent une activité dans le domaine de l'immunosuppression supérieure aux produits connus de l'art antérieur.
Les produits selon l'invention se distinguent notamment des produits connus de l'art antérieur, du point de vue de la structure chimique, par l'inversion de la liaison CO-NH liant le reste guanidinehexyle ou guanidineoctyle à l'aminoacide central.
Les composés analogues de la 15-déoxyspergualine selon l'invention sont caractérisés en ce qu'ils sont choisis parmi l'ensemble constitué par :

(i) les composés de formule : 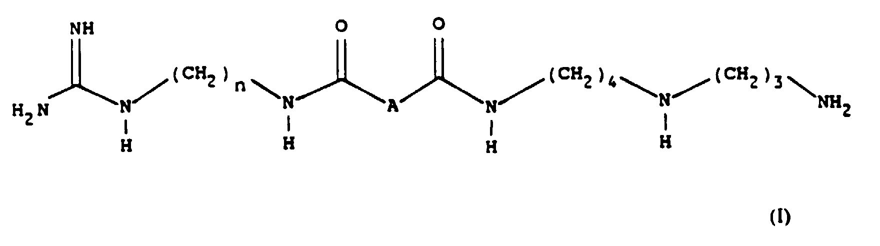 dans laquelle :
   - n est égal à 6 ou 8,
   - A représente une simple liaison, un groupe CH₂, un groupe CH(OH), un groupe CHF, un groupe CH(OCH₃), un groupe CH₂NH ou un groupe CH₂O, et,
(ii) leurs sels d'addition.

Selon l'invention, on préconise également un procédé de préparation des composés de formule I et de leurs sels d'addition, ledit procédé étant caractérisé en ce qu'il comprend la déprotection d'un composé de formule : 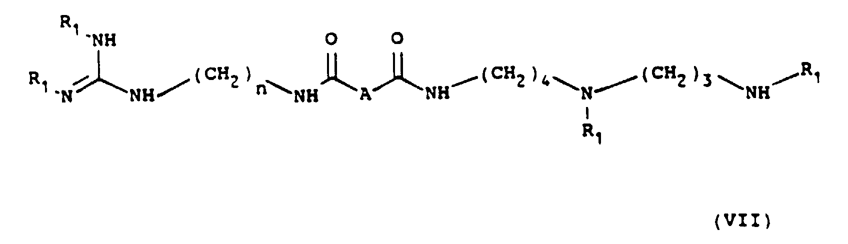 dans laquelle n et A sont définis comme indiqué ci-dessus, et R₁ représente un groupe protecteur de fonction amine, notamment au moyen d'un acide fort pour le remplacement de R₁ par H.

On préconise également l'utilisation d'une substance immunosuppressive choisie parmi l'ensemble constitué par les composés de formule I et leurs sels d'addition non toxiques, pour l'obtention d'un médicament destiné à une utilisation en thérapeutique vis-à-vis des désordres immunitaires.

On préconise enfin l'utilisation d'une substance choisie parmi l'ensemble constitué par les composés de formule I et leurs sels d'addition non toxiques, pour l'obtention de médicaments destinés au traitement du paludisme.
Bien entendu, dans de telles utilisations l'ingrédient actif interviendra suivant une quantité thérapeutiquement efficace.

### DESCRIPTION DETAILLEE DE L'INVENTION

Par sels d'addition, on entend les sels d'addition d'acides obtenus par réaction d'un acide minéral ou d'un acide organique avec un composé de formule I. Les acides minéraux préférés pour la salification sont les acides chlorhydrique, bromhydrique, sulfurique et phosphorique. Les acides organiques préférés pour la salification sont les acides fumarique, maléique, méthanesulfonique, oxalique, citrique et trifluoroacétique.

Les composés de formule I peuvent être préparés selon des méthodes connues en soi par application de mécanismes réactionnels classiques, comme la formation d'une liaison amide et notamment en applicant les méthodes connues de la chimie des peptides.

Le procédé de préparation que l'on préconise suivant l'invention comprend, comme indiqué ci-dessus, la déprotection d'un composé de formule VII.
De façon pratique, chaque groupe protecteur R₁, que l'on va remplacer par un atome d'hydrogène, sera un groupe de type oxycarbonyle connu dans le domaine de la synthèse peptidique pour bloquer momentanément les fonctions "amino" non totalement substituées. Parmi les groupes protecteurs qui conviennent à cet effet, on peut mentionner les restes donnés ci-après avec leurs abréviations classiques :
- Adoc: = adamantyloxycarbonyle
- Aoc: = t-amyloxycarbonyle
- Boc: = t-butyloxycarbonyle (autre nomenclature : (1,1 -diméthyléthoxy)carbonyle)
- Fmoc: = 9-fluorènylméthyloxycarbonyle
- Foc: = furfuryloxycarbonyle
- Iboc: = isobornyloxycarbonyle
- Z: = benzyloxycarbonyle
- Z(p-Cl): = p-chlorobenzyloxycarbonyle
- Z(p-OMe): = p-méthoxybenzyloxycarbonyle

Parmi ces groupes, appelés dans la suite du texte "amino-protecteurs", le groupe R₁ que l'on préfère selon l'invention est le groupe Boc.

De façon également pratique, le procédé de préparation d'un composé de formule I ou de l'un de ses sels d'addition est caractérisé en ce qu'il est choisi parmi l'ensemble constitué par :
- la variante A, qui comprend les étapes consistant à :
   (i) faire réagir un composé de formule : 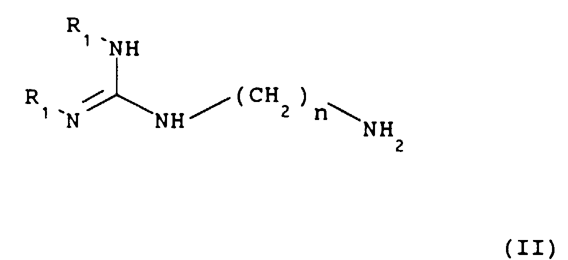 dans laquelle n est égal à 6 ou 8 et R₁ représente un groupe amino-protecteur, notamment le groupe (1,1-diméthyl-éthoxy)carbonyle, avec un acide ou un chlorure d'acide de formule : 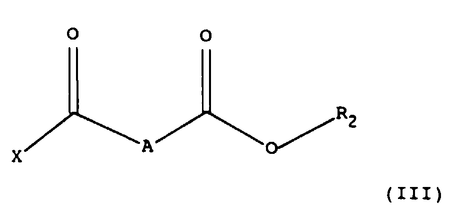 dans laquelle :
      - X représente un atome de chlore ou un groupe OH,
      - A représente une simple liaison, un groupe CH₂, un groupe CHF, un groupe CH(OCH₂C₆H₅) ou un groupe CH(OCH₃), et,
      - R₂ représente un groupe alkyle en C₁-C₃, linéaire ou ramifié, ou un groupe phénylméthyle,
      dans un solvant organique (notamment un solvant chloré, comme par exemple le dichlorométhane ou le chloroforme), en présence d'un activateur de groupe carboxylique (notamment un carbodiimide, comme par exemple le 1,3-dicyclohexylcarbodiimide) et en présence d'un agent nucléophile (notamment le 1-hydroxybenzotriazole), à une température comprise entre 0°C et environ 40°C, à raison d'environ 1 mole de II pour environ 1 mole de III, pour obtenir un composé de formule : 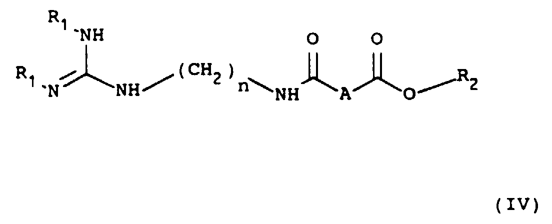 dans laquelle R₁, R₂ et n ont les significations indiquées ci-dessus, et A représente une simple liaison, CH₂, CHF, CH(OCH₂C₆H₅) ou CH(OCH₃),
   (ii) saponifier le composé de formule IV, ainsi obtenu, dans un solvant organique en présence d'une base forte pour obtenir un composé de formule : 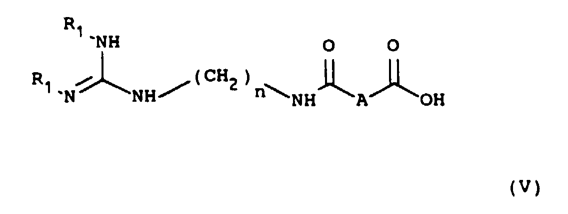 dans laquelle R₁ et n ont les significations indiquées ci-dessus, et A représente une simple liaison, CH₂, CHF, CH(OCH₂C₆H₅) ou CH(OCH₃),
   (iii) condenser le composé de formule V, ainsi obtenu, sur une amine de formule : 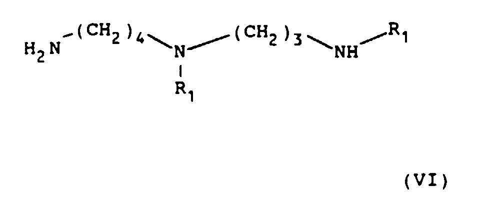 dans laquelle R₁ a la signification indiquée ci-dessus, dans des conditions identiques à celles de l'étape (i) ci-dessus, pour obtenir un composé de formule : 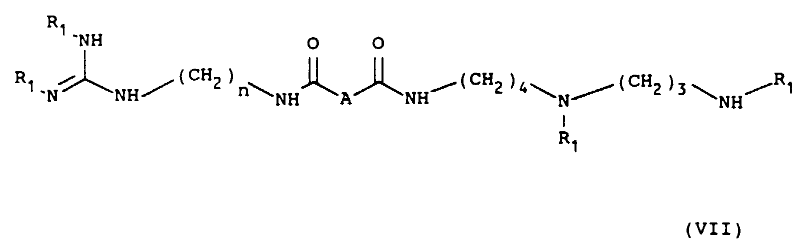 dans laquelle R₁ et n ont les significations indiquées ci-dessus, et A représente une simple liaison, CH₂, CHF, CH(OCH₂C₆H₅) ou CH(OCH₃),
   (iv) si nécessaire, déprotéger le composé de formule VII dans laquelle A représente le groupe CH(OCH₂C₆H₅), par hydrogénation catalytique, pour obtenir le composé de formule VII dans laquelle A représente le groupe CH(OH),
   (v) déprotéger le composé VII obtenu à l'étape (iii) ou (iv), où A représente une simple liaison, CH₂, CHF, CH(OH) ou CH(OCH₃), pour éliminer le groupe protecteur R₁, notamment par action d'un acide fort comme par exemple l'acide trifluoroacétique, et obtenir ainsi un sel d'addition d'un composé de formule I, où A est une simple liaison, CH₂, CHF, CH(OH) ou CH(OCH₃), et
   (vi) si nécessaire, obtenir ledit composé de formule I sous forme de base libre par action d'une base forte, puis à partir de ladite base libre les autres sels d'addition ;
- la variante B, qui comprend les étapes consistant à :
   (i) faire réagir un composé de formule : 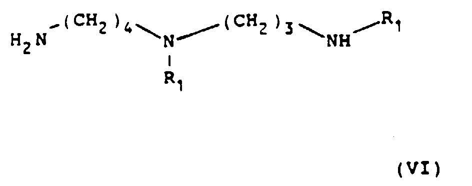 dans laquelle R₁ représente un groupe amino-protecteur comme indiqué ci-dessus [notamment (1,1-diméthyléthoxy)carbonyle], avec un acide ou un chlorure d'acide de formule : 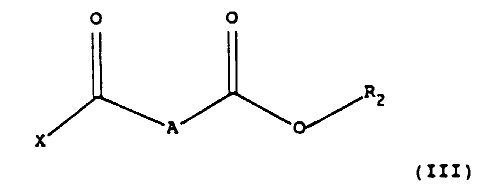 dans laquelle :
      - X représente un atome de chlore ou un groupe OH,
      - A représente une simple liaison, un groupe CH₂ un groupe CH(OCH₂C₆H₅), un groupe CH(OCH₃), ou un groupe CHF et,
      - R₂ représente un groupe alkyle en C₁-C₃, linéaire ou ramifié, ou un groupe phénylméthyle,
      dans un solvant organique (notamment un solvant chloré, comme par exemple le dichlorométhane ou le chloroforme), en présence d'un activateur de groupe carboxylique (notamment un carbodiimide, comme par exemple le 1,3-dicyclohexylcarbodiimide) et en présence d'un agent nucléophile notamment le 1-hydroxybenzotriazole), à une température comprise entre 0°C et environ 40°C, à raison d'environ 1 mole de VI pour environ 1 mole de III, pour obtenir un composé de formule : 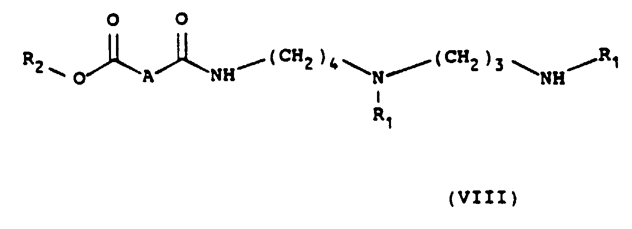 dans laquelle R₁ et R₂ ont les significations indiquées ci-dessus, et A représente une simple liaison, CH₂, un groupe CH (OCH₂C₆H₅), un groupe CH(OCH₃), ou un groupe CHF,
   (ii) saponifier le composé de formule VIII, ainsi obtenu, dans un solvant organique en présence d'une base forte pour obtenir un composé de formule : 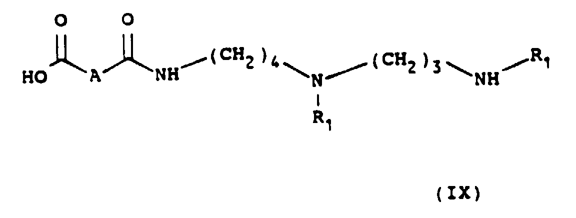 dans laquelle R₁ a les significations indiquées ci-dessus, et A représente une simple liaison, CH₂, un groupe CH(OCH₂C₆H₅), un groupe CH(OCH₃), ou un groupe CHF,
   (iii) condenser le composé de formule IX, ainsi obtenu, sur une amine de formule : 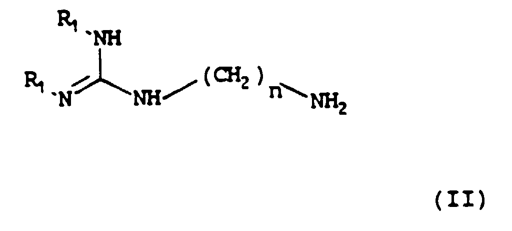 dans laquelle n est égal à 6 ou 8, et R₁ est défini comme indiqué ci-dessus,
      dans des conditions identiques à celles de l'étape (i) ci-dessus, pour obtenir un composé de formule : 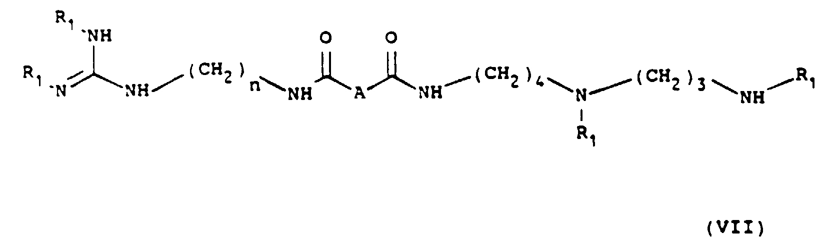 dans laquelle R₁ et n sont définis comme indiqué ci-dessus et A représente une simple liaison, CH₂, un groupe CH(OCH₂C₆H₅), un groupe CH(OCH₃), ou un groupe CHF,
   (iv) si nécessaire, déprotéger le composé de formule VII dans laquelle A représente le groupe CH(OCH₂C₆H₅), par hydrogénation catalytique, pour obtenir le composé de formule VII dans laquelle A représente le groupe CH(OH),
   (v) déprotéger le composé VII, ainsi obtenu, par action d'un acide fort (notamment l'acide trifluoroacétique) pour obtenir un sel d'addition d'un composé de formule I, où A est une simple liaison, CH₂, CH(OH), CH(OCH₃) ou un groupe CHF,
   (vi) si nécessaire, obtenir ledit composé de formule I sous forme de base libre par action d'une base forte, puis à partir de ladite base libre les autres sels d'addition ;
- la variante C qui comprend les étapes consistant à :
   (i) acyler l'extrémité NH₂-terminale d'une base de formule : 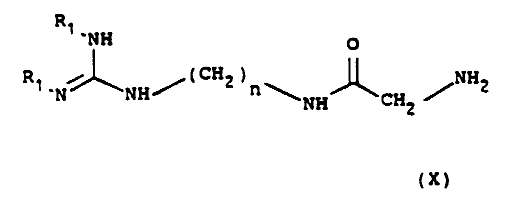 dans laquelle R₁ représente un groupe protecteur, comme indiqué ci-dessus [notamment (1,1-diméthyléthoxy)carbonyle] et n est égal à 6 ou 8, par un chloroformiate ou un carbonate symétrique [notamment le bis(4-nitrophényl)carbonate], dans un solvant inerte, à la température ambiante (15-25°C),
   (ii) aminolyser le composé, ainsi obtenu, par une amine de formule : 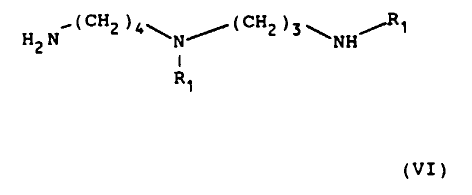 dans laquelle R₁ a la signification indiquée ci-dessus,
      pour obtenir un composé de formule : 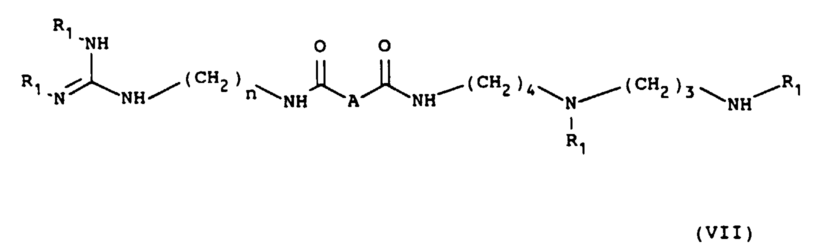 dans laquelle R₁ et n ont les significations indiquées ci-dessus, et A représente le groupe CH₂NH,
   (iii) déprotéger le composé VII, ainsi obtenu, par action d'un acide fort (notamment l'acide trifluoroacétique), pour obtenir un sel d'addition d'un composé de formule I où A est CH₂NH, et
   (iv) si nécessaire, obtenir ledit composé de formule I sous forme de base libre par action d'une base forte, puis à partir de ladite base libre les autres sels d'addition ; et
- la variante D qui comprend les étapes consistant à :
   (i) faire réagir un composé de formule : 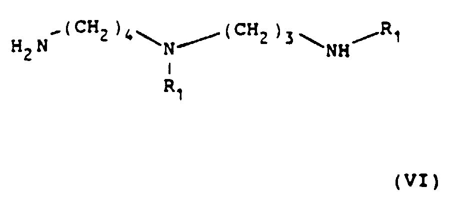 dans laquelle R₁ représente un groupe amino-protecteur, comme indiqué ci-dessus [notamment (1,1-diméthyléthoxy)carbonyle], avec un carbonate de formule : 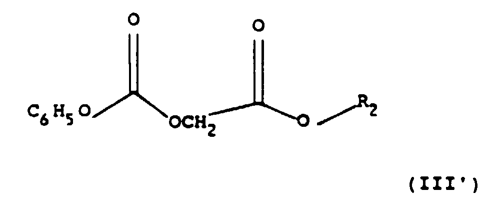 dans laquelle :
      - R₂ représente un groupe alkyle en C₁-C₃, linéaire ou ramifié, ou un groupe phénylméthyle,
      dans un solvant organique inerte (notamment un solvant aromatique comme par exemple le toluène), à la température de reflux du milieu réactionnel, à raison d'environ 1 mole de VI pour environ 1 mole de III', pour obtenir un composé de formule : 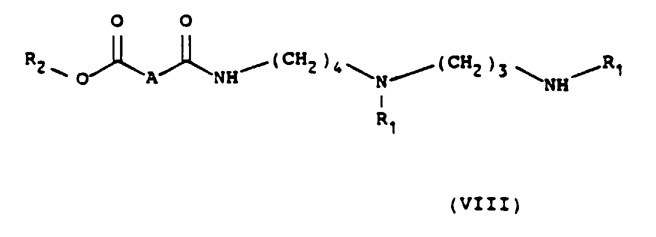 dans laquelle R₁ et R₂ ont les significations indiquées ci-dessus et A représente le groupe CH₂O,
   (ii) saponifier le composé de formule VIII, ainsi obtenu, dans un solvant organique en présence d'une base forte pour obtenir un composé de formule : 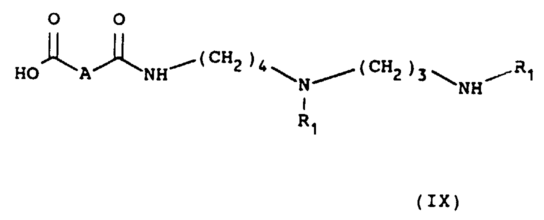 dans laquelle R₁ a les significations indiquées ci-dessus, et A représente CH₂O,
   (iii) condenser le composé de formule IX, ainsi obtenu, sur une amine de formule : 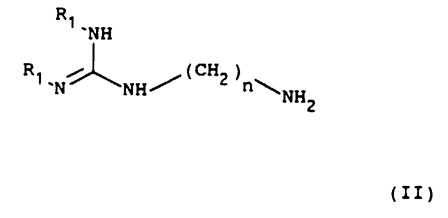 dans laquelle R₁ est défini comme indiqué ci-dessus, et n est égal à 6 ou 8, dans un solvant organique (notamment un solvant chloré comme par exemple le dichlorométhane ou le chloroforme), en présence d'un activateur de groupe carboxylique (notamment un carbodiimide, comme par exemple le 1,3-dicyclohexylcarbodiimide) et en présence d'un agent nucléophile (notamment le 1-hydroxybenzotriazole), à une température comprise entre 0°C et environ 40°C, à raison d'environ 1 mole de IX pour environ 1 mole de II, pour obtenir un composé de formule : 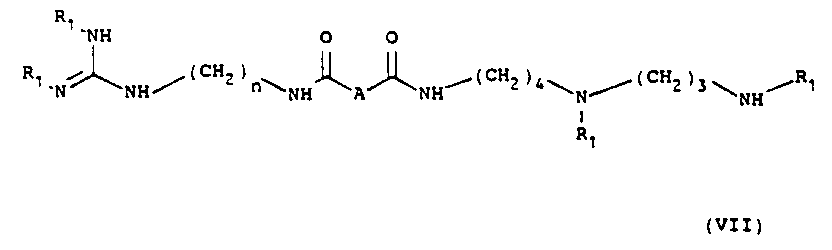 dans laquelle R₁ et n sont définis comme indiqué ci-dessus et A représente CH₂O,
   (iv) déprotéger le composé VII, ainsi obtenu, par action d'un acide fort (notamment l'acide trifluoroacétique), pour obtenir un sel d'addition d'un composé de formule I où A est CH₂O, et
   (v) si nécessaire, obtenir ledit composé de formule I sous forme de base libre par action d'une base forte, puis à partir de ladite base libre les autres sels d'addition.

Le composé VI où R₁ est un groupe (1,1-diméthyl-éthoxy)carbonyle peut être obtenu selon la méthode préconisée par Raymond J. BERGERON, "Total Synthesis of (±)-15-Deoxyspergualin", J. Org. Chem. 1987, 52,1700-1703.

On peut également obtenir les composés de formule IV par génération puis aminolyse d'un anhydride mixte : on fait réagir l'acide de formule III avec un chloroformiate, notamment le chloroformiate d'isobutyle, en présence d'un équivalent d'une base tertiaire, notamment la N-méthylmorpholine, dans un solvant non réactif, à une température d'environ - 30°C, pendant environ 0,5 heure, puis on additionne au milieu réactionnel la base Il.

La formation d'une liaison amide peut également être réalisée selon les méthodes connues de l'homme de l'art, notamment par acylation d'une amine appropriée par un chlorure d'acide dans un solvant non réactif, en présence d'une base organique forte.

Les composés intermédiaires de formule VII dans laquelle R₁ représente un groupe amino-protecteur, notamment du type oxycarbonyle, n est égal à 6 ou 8 et A représente une simple liaison, un groupe CH₂, un groupe CH(OH), un groupe CH(OCH,), un groupe CH₂NH, un groupe CH₂O, un groupe CH(OCH₂C₆H₅) ou un groupe CHF sont des composés nouveaux et constituent un autre objet de l'invention.

L'invention sera mieux comprise à la lecture des exemples qui suivent et des résultats pharmacologiques obtenus avec les composés selon l'invention comparativement aux résultats obtenus avec les produits connus de l'art antérieur. La nomenclature utilisée ci-après est celle préconisée par les Chemical Abstracts ; selon cette nomenclature un diester du type alcanedioate de t-butyle et d'éthyle est dénommé ici "acide alcanedioïque, (1,1-diméthyléthyl) éthyl ester".

### PREPARATION I

### Acide [[[6-aminohexyl)imino]méthylène]bis-carbamique, bis-(1,1-diméthyl éthyl) ester.

Sous agitation et à température ambiante, on ajoute 17,23 g (0,148 mole) de 1,6-hexanediamine à une solution de 43 g (0,148 mole) de N,N'-bis(tert-butoxycarbonyl)-S-méthylisothiourée dans 300 ml de tétrahydrofuranne. On agite le milieu réactionnel pendant 16 heures. Après évaporation du solvant, le résidu obtenu est chromatographié sur silice en éluant avec un mélange CHCl₃/éthanol 3/1 (v/v) puis avec un mélange acétate d'éthyle/méthanol/ammoniaque à 32 % dans l'eau 6/3/0,1 (v/v/v). On obtient ainsi 19,7 g (rendement : 37 %) d'une huile jaune.
**RMN** ^{**1**}**H (CDCl**_{**3**}**) : 1,25-1,60 (m,28H) ; 2,7 (t,2H) ; 3,5 (q,2H) ; 8,3 (t,1H) ; 11,5 (s,1H).**

En opérant de manière analogue, on obtient le produit suivant :
**Acide [[[8-aminooctyl)imino]méthylène]bis-carbamique, bis-(1,1-diméthyl éthyl) ester.**
**RMN** ^{**1**}**H (CDCl**_{**3**}**) : 1,3-1,7 (m,32H) ; 2,7 (t,2H) ; 3,4 (q,2H) ; 8,3 (t,1H) ; 11,5 (s,1H).**

### PREPARATION II

### Acide 6-[(1,1-diméthyléthoxylcarbonyl]-12-oxo-2,6,11-triazatétradécane dioïque, 1-(1,1-diméthyléthyl) 14-éthyl ester.

On ajoute 0,82 g 14.10⁻³ moles) de 1,3-dicyclohexylcarbodiimide à une solution refroidie à 0°C de 0,53 g (4.10⁻³ moles) de malonate d'éthyle dans 20 ml de chloroforme anhydre. Après 0,5 heure d'agitation, on additionne goutte à goutte et à 0°C, 1,04 g (3.10⁻³ moles) d'acide 10-amino-6-[(1,1-diméthyléthoxy)carbonyl]-2,6-diazadécanoïque, 1,1-diméthyléthyl ester en solution dans 5 ml de chloroforme anhydre. On agite pendant 5 heures à température ambiante et on additionne 1,06 g (8.10⁻³ moles) de malonate d'éthyle et 1,64 g (8.10⁻³ moles) de 1,3-dicyclohexylcarbodiimide. On agite pendant 1 heure et on évapore le solvant sous pression réduite. Le résidu pâteux obtenu est chromatographié sur silice en éluant avec un mélange acétate d'éthyle/hexane 1/1 (v/v) puis à l'acétate d'éthyle. On obtient 0,95 g (rendement : 69 %) du produit attendu sous forme d'huile.
**RMN** ^{**1**}**H (CDCl**_{**3**}**) : 1,25 (t,3H) ; 1,40-1,70 (m,24H) ; 3,10-3,35 (m,10H) ; 4,2 (q,2H).**

### PREPARATION III

### Acide 6-[(1,1-diméthyléthoxy)carbonyl]-12-oxo-2,6,11-triazatétradécane dioïque, 1-(1,1-diméthyléthyl) ester.

0,95 g (2,07.10⁻³ moles) du produit obtenu à la préparation Il sont dissous dans un mélange de 20 ml de soude N et 20 ml de diméthoxyéthane 1/1 (v/v). On agite 15 minutes à température ambiante et on réduit d'un tiers le volume du mélange réactionnel que l'on acidifie ensuite jusqu'à un pH compris entre 2 et 3 par de l'acide chlorhydrique 1N. On extrait ensuite par 2 fois 50 ml de chloroforme. Après évaporation sous pression réduite des phases organiques, le résidu obtenu est chromatographié sur silice en éluant avec un mélange acétate d'éthyle/méthanol 3/1 (v/v) puis au méthanol. On obtient ainsi 0,75 g (rendement : 84 %) du produit attendu sous forme d'huile.
**RMN** ^{**1**}**H (diméthylsulfoxyde-d6) : 1,40 (s,18H) ; 1,55 (m,6H) ; 2,90 (m,4H) ; 3,15 (m,6H).**

### PREPARATION IV

### Acide 3-[[(1,1-diméthyléthoxy)carbonyl]amino]-20-[(1,1-diméthyléthoxy) carbonyl]-12,14-dioxo-2,4,11,15,20,24-hexaazapentacos-2-ènedioïque, bis-(1,1-diméthyléthyl) ester.

A une solution à 0°C de 0,5 g (1,16.10⁻³ mole) du produit obtenu à la préparation III dans 30 ml de chloroforme anhydre, on ajoute sous agitation 0,46 g (2,32.10⁻³ moles) de 1,3-dicyclohexylcarbodiimide et 0,0155 g (0,1.10⁻³ mole) d'hydrate de 1-hydroxybenzotriazole. Après agitation du milieu réactionnel pendant 0,5 heure, on ajoute goutte à goutte et à 0°C 0,42 g (1,16.10⁻³ mole) d'acide [[(6-aminohexyl)iminolméthylènelbis-carbamique, bis(1,1-diméthyléthyl) ester (obtenu selon le procédé de la préparation I ci-dessus). On agite à nouveau le milieu réactionnel à 0°C pendant 1,5 heure puis on ajoute 0,23 g (1,16.10⁻³ mole) de 1,3-dicyclohexylcarbodiimide et on poursuit l'agitation pendant 24 heures à température ambiante. On évapore le solvant sous pression réduite et le résidu obtenu est chromatographié sur silice en éluant avec un mélange acétate d'éthyle/hexane 1/1 (v/v) puis à l'acétate d'éthyle et enfin avec un mélange acétate d'éthyle/méthanol 9/1 (v/v). On obtient ainsi 0,7 g (rendement : 73 %) du produit attendu sous forme d'huile.
**RMN** ^{**1**}**H (CDCl**_{**3**}**) : 1,3-1,7 (m,50H) ; 3,1-3,3 (m,12H) ; 3,4 (q,2H) ; 4,8 et 5,3 (s large,1H) ; 6,8 et 7,15 (s large,2H) ; 8,3 (t,1H) ; 11,5 (s,1H).**

### Exemple 1

### N-[4-[[3-(amino)propyl]amino]butyl]-N'-[6-[(aminoiminométhyl)amino] hexyl]-propanediamide, tris-(trifluoroacétate).

On dissout 0,7 g (0,9.10⁻³ mole) du produit obtenu à la préparation IV dans 10 ml d'acide trifluoroacétique et 10 ml de dichlorométhane anhydre. On agite le milieu réactionnel pendant 24 heures à température ambiante puis on évapore le solvant sous pression réduite. Le résidu obtenu est repris par 150 ml d'eau distillée puis lyophilisé. Le résidu est purifié par MPLC (chromatographie liquide moyenne pression) sur phase inverse (silice RP 18) en éluant avec un mélange eau/acétonitrile/acide trifluoroacétique 7/2/1 (v/v/v). On obtient 0,43 g (rendement : 66 %) d'un solide très hygroscopique.
**RMN** ^{**1**}**H (diméthylsulfoxyde-d6) : 1,2-1,6 (m,12H) ; 1,9 (m,2H) ; 2,9-3,1 (m,14H) ; 7,2 (s large,4H) ; 7,7 (t,1H) ; 8 (m,5H) ; 8,7 (s large,2H).**
**RMN** ^{**13**}**C (diméthylsulfoxyde-d6) : 22,5 ; 23,4 ; 25,4 ; 25,6 ; 25,7 ; 28,0 ; 28,5 ; 35,8 ; 37,6 ; 38,2 ; 40,3 ; 43,0 ; 43,5 ; 46,1; 156,6 ; 166,9 (2C).**

### PREPARATION V

### Acide 3-[[(1,1-diméthyléthoxy)carbonyl]amino]-14-oxo-2,4,13-triazahexa déc-2-ènedioïque, 1-(1,1-diméthyléthyl) 16-éthyl ester.

En opérant de façon analogue à la préparation IV et au départ de 1,32 g (10.10⁻³ moles) de malonate d'éthyle et 2 g (5,18.10⁻³ moles) d'acide [[(8-amino-octyl)imino]méthylène]bis-carbamique, bis(1,1-diméthyléthyl) ester, on obtient 1,96 g (rendement : 75 % ) du produit attendu sous forme d'huile.
**RMN** ^{**1**}**H (CDCl**_{**3**}**) : 1,25-1,70 (m,33H) ; 3,25 (q,2H) ; 3,30 (s,2H) ; 3,40 (q,2H); 4,2 (q,2H) ; 7,25 (s,1H) ; 8,3 (s,1H) ; 11,5 (s,1H).**

### PREPARATION VI

### Acide 3-[[(1,1-diméthyléthoxy)carbonyl]amino]-14-oxo-2,4,13-triazahexa déc-2-ènedioïque, 1-(1,1-diméthyléthyl) ester.

En opérant de façon analogue à la préparation III et au départ de 1,96 g (3,92.10⁻³ moles) du produit obtenu à la préparation V, on obtient 1,92 g (rendement : 100 %) du produit attendu sous forme d'huile.
**RMN** ^{**1**}**H (CDCl**_{**3**}**) : 1,25-1,70 (m,30H) ; 3,27-3,39 (m,6H) ; 7,25 (s,1H) ; 8,40 (s,1H).**

### PREPARATION VII

### Acide 3-[[(1,1-diméthyléthoxy)carbonyl]amino]-22-[(1,1-diméthyléthoxy) carbonyl]-14,16-dioxo-2,4,13,17,22,26-hexaazaheptacos-2-ènedioïque, bis-(1,1-diméthyléthyl) ester.

En opérant de façon analogue à la préparation IV et au départ de 1,85 g (3,92.10⁻³ moles) du produit obtenu à la préparation VI et de 1,35 g (3,92.10⁻³ moles) d'acide 10-amino-6-[(1,1-diméthyléthoxy)carbonyl]-2,6-diazadécanoïque, 1,1-(diméthyléthyl) ester, on obtient 0,41 g (rendement : 13 %) du produit attendu sous forme d'huile.
**RMN** ^{**1**}**H (CDCl**_{**3**}**) : 1,30-1,70 (m,54H) ; 3,1-3,5 (m,14H) ; 4,8 et 5,3 (s large,1H)) ; 6,7 et 7,1 (s large,2H) ; 8,3 (t,1H) ; 11,5 (s,1H).**

### Exemple 2

### N-[4-[[3-(amino)propyl]amino]butyl]-N'-[8-[(aminoiminométhyl)amino] octyl]-propanediamide, tris-(trifluoroacétate).

En opérant de façon analogue au procédé de l'exemple 1 et au départ de 0,41 g (0,51.10⁻³ mole) du produit obtenu à la préparation VII, on obtient 0,24 g (rendement : 65 %) du produit attendu sous forme d'huile.
**RMN** ^{**1**}**H (diméthylsulfoxyde-d6) : 1,2-1,6 (m,16H) ; 1,9 (m,2H) ; 2,9-3,1 (m,14H) ; 7,2 (s large,4H) ; 7,7 (t,1H) ; 8 (m,5H) ; 8,7 (s large,2H).**
**RMN** ^{**13**}**C (diméthylsulfoxyde-d6) : 22,9 ; 23,7 ; 26,0 ; 26,1 ; 26,3 ; 28,4 ; 28,5 ; 28,6 ; 28,9 ; 36,2 ; 37,9 ; 38,6 ; 40,7 ; 43,3 ; 43,8 ; 46,4 ; 156,8; 166,7 ; 166,9.**

### PREPARATION VIII

### Acide 3-[[(1,1-diméthyléthoxy)carbonyl]amino]-13-méthoxy-12-oxo-2,4,11-triazatétradéc-2-ènedioïque, 1-(1,1-diméthyléthyl) 14-méthyl ester.

En opérant de façon analogue à la préparation IV et au départ de 1,8 g (12.10⁻³ moles) d'acide 2-méthoxy-propanedioïque, méthyl ester et de 4 g (11.10⁻³ moles) d'acide [[(6-aminohexyl)imino]méthylène]bis-carbamique, bis(1,1-diméthyléthyl) ester et en remplaçant le chloroforme par du dichlorométhane, on obtient 4,8 g (rendement : 87 %) du produit attendu sous forme d'huile.
**RMN** ^{**1**}**H (CDCl**_{**3**}**) : 1,35-1,65 (m,26H) ; 3,2 (m,2H) ; 3,35 (q,2H) ; 3,45 (s,3H) ; 3,8 (s,3H) ; 4,3 (s,1H) ; 6,7 (t,1H) ; 8,3 (t,1H) ; 11,5 (s,1H).**

### PREPARATION IX

### Acide 3-[[(1,1-diméthyléthoxy)carbonyl]amino]-13-méthoxy-12-oxo-2,4,1-triazatétradéc-2-ènedioïque, 1-(1,1-diméthyléthyl) ester.

En opérant de façon analogue à la préparation III et au départ de 4,6 g (9,4.10⁻³ moles) du produit obtenu à la préparation VIII, on obtient 4 g (rendement : 89 %) du produit attendu sous forme d'huile.
**RMN** ^{**1**}**H (CDCl**_{**3**}**) : 1,3-1,65 (m,26H) ; 3,1-3,5 (m,4H) ; 3,6 (s,3H) ; 4,3 (s,1H) ; 6,7 (t,1H) ; 8,4 (t,1H) ; 11,5 (s large,1H).**

### PREPARATION X

### Acide 3-[[(1,1-diméthyléthoxy)carbonyl]amino]-20-[(1,1-diméthyléthoxy) carbonyl]-13-méthoxy-12,14-dioxo-2,4,11,15,20,24-hexaazapentacos-2-ènedioïque, bis-(1,1-diméthyléthyl) ester.

En opérant de façon analogue à la préparation IV et au départ de 3,77 g (8.10⁻³ moles) du produit obtenu à la préparation IX et de 2,7 g (8.10⁻³ moles) d'acide 10-amino-6-[(1,1-diméthyléthoxy)carbonyl]-2,6-diazadécanoïque, 1,1-(diméthyléthyl) ester et en remplaçant le chloroforme par du dichlorométhane, on obtient 4 g (rendement : 63 %) du produit attendu sous forme d'huile.
**RMN** ^{**1**}**H (CDCl**_{**3**}**) : 1,3-1,8 (m,50H) ; 3,0-3,45 (m,12H) ; 3,6 (s,3H) ; 4,1 (m,1H) ; 4,8 et 5,3 (s large,1H) ; 6,9 (s large,2H) ; 8,3 (t,1H) ; 11,5 (s,1H).**

### Exemple 3

### N-[4-[[3-(amino)propyl]amino]butyl]-N'-[6-[(aminoiminométhyl)amino] hexyl]-2-méthoxy-propanediamide, tris-(trifluoroacétate).

En opérant de façon analogue au procédé de l'exemple 1 et au départ de 3,47 g (4,3.10⁻³ moles) du produit obtenu à la préparation X, on obtient 2,86 g (rendement : 89 %) du produit attendu sous forme d'huile.
**RMN** ^{**1**}**H (diméthylsulfoxyde-d6) : 1,25-1,70 (m,12H) ; 1,9 (m,2H) ; 2,85-3,2 (m,12H) ; 3,25 (s,3H) ; 4,1 (s,1H) ; 6,8-8,7 (m,12H).**
**RMN** ^{**13**}**C (CD**_{**3**}**OD) : 24.2 ; 25,3 ; 27,36 ; 27,37 ; 29,9 ; 30,2 ; 37,9 ; 39,2 ; 40,2 ; 42,5 ; 45,7 ; 58,6 ; 158,7 ; 169,4 ; 169,8.**

### PREPARATION XI

### Acide 6-[(1,1-diméthyléthoxy)carbonyl]-13-méthoxy-12-oxo-2,6,11-triaza tétradécanedioïque, 1-(1,1-diméthyléthyl) 14-méthyl ester.

En opérant de façon analogue à la préparation IV et au départ de 2,62 g (17,7.10⁻³ moles) d'acide 2-méthoxypropanedioïque, méthyl ester et de 3,45 g (10.10⁻³ moles) d'acide 10-amino-6-[(1,1-diméthyl-éthoxy) carbonyl]-2,6-diazadécanoïque, 1,1-(diméthyl-éthyl) ester, on obtient 1,5 g (rendement : 18 %) du produit attendu sous forme d'huile.
**RMN** ^{**1**}**H (CDCl**_{**3**}**) : 1,4-1,8 (m,24H) ; 3,1-3,4 (m,8H) ; 3,5 (s,3H) ; 3,8 (s,3H) ; 4,3 (s,1H) ; 4,8 et 5,3 (s large,1H) ; 6,7 (s,1H).**

### PREPARATION XII

### Acide 6-[(1,1-diméthyléthoxy)carbonyl]-13-méthoxy-12-oxo-2,6,11-triaza tétradécanedioïque, 1-(1,1-diméthyléthyl) ester.

En opérant de façon analogue à la préparation III et au départ de 1,5 g (3,16.10⁻³ moles) du produit obtenu à la préparation XI, on obtient 1,18 g (rendement : 81 %) du produit attendu sous forme d'huile.
**RMN** ^{**1**}**H (CDCl**_{**3**}**) : 1,4-1,8 (m,24H) ; 3,15-3,35 (m,8H) ; 3,5 (s,3H) ; 4,3 (s,1H) ; 5,2-5,7 (s double,1H) ; 6,3-6,9 (s double,1H).**

### PREPARATION XIII

### Acide 3-[[(1,1-diméthyléthoxy)carbonyl]amino]-22-[(1,1-diméthyléthoxy) carbonyl]-15-méthoxy-14,16-dioxo-2,4,13,17,22,26-hexaazaheptacos-2-ènedioïque, bis-(1,1-diméthyléthyl) ester.

En opérant de façon analogue à la préparation IV et au départ de 1,18 g (2,56.10⁻³ moles) du produit obtenu à la préparation XII, on obtient 1,4 g (rendement : 65 %) du produit attendu sous forme d'huile.
**RMN** ^{**1**}**H (CDCl**_{**3**}**) : 1,3-1,8 (m,54H) ; 3,1-3,7 (m,15H) ; 4,1 (s,1H) ; 4,8 et 5,3 (s large,1H) ; 6,9 (s large,2H) ; 8,3 (t,1H) ; 11,5 (s,1H).**

### Exemple 4

### N-[4-[[3-(amino)propyl]amino]butyl]-2-méthoxy-N'-[8-[(aminoimino méthyl)amino]octyl]-propanediamide, tris-(trifluoroacétate).

En opérant de façon analogue au procédé de l'exemple 1 et au départ de 1,37 g (1,65.10⁻³ mole) du produit obtenu à la préparation XIII, on obtient 0,89 g (rendement : 70 %) du produit attendu sous forme d'huile.
**RMN** ^{**1**}**H (diméthylsulfoxyde-d6) : 1,2-1,7 (m,16H) ; 1,9 (m,2H) ; 2,9-3,10 (m,12H) 3,3 (s,3H) ; 4,1 (s,1H) ; 6,8-8,7 (m,12H).**
**RMN** ^{**13**}**C (D**_{**2**}**O) : 23,8 ; 25,0 ; 26,5 ; 27,0 ; 27,3 ; 28,5 ; 29,1 (2C) ; 37,5 ; 39,8 ; 40,2 ; 42,5 ; 45,1 ; 48,2 ; 58,4 ; 63,9 ; 82,5 ; 157.8 ; 169,8 ; 170,2.**

### PREPARATION XIV

### Acide 6-[(1,1-diméthyléthoxy)carbonyl]-13-oxa-12-oxo-2,6,11-triaza pentadécanedioïque, 1-(1,1-diméthyléthyl) 15-méthyl ester.

A une solution de 4,5 g (21,4.10⁻³ moles) d'acide [(phénoxy carbonyl)oxy]-acétique, méthyl ester dans 100 ml de toluène, on ajoute sous agitation 7,4 g (21,4.10⁻³ moles) d'acide 10-amino-6-[(1,1-diméthyléthoxy)carbonyl]-2,6-diazadécanoïque, 1,1-(diméthyléthyl) ester en solution dans 20 ml de toluène. On porte le milieu réactionnel au reflux pendant 15 heures. On évapore le solvant sous pression réduite et le résidu obtenu est purifié par chromatographie sur silice en éluant avec un mélange méthylcyclohexane/acétate d'éthyle 7/3 (v/v) puis à l'acétate d'éthyle. On obtient ainsi 8,4 g (rendement : 85 %) du produit attendu sous forme d'huile.
**RMN** ^{**1**}**H (CDCl**_{**3**}**) : 1,45-1,65 (m,24H) ; 3,05-3,25 (m,8H) ; 3,8 (s,3H) ; 4,7 (s,2H) ; 4,9 et 5,3 (s large,1H).**

### PREPARATION XV

### Acide 6-[(1,1-diméthyléthoxy)carbonyl]-13-oxa-12-oxo-2,6,11-triaza pentadécanedioïque, 1-(1,1-diméthyléthyl) ester.

En opérant de façon analogue à la préparation III et au départ de 8,45 g 118,3.10⁻³ moles) du produit obtenu à la préparation XIV, on obtient 6,7 g (rendement : 81 %) du produit attendu sous forme d'huile.
**RMN** ^{**1**}**H (CDCl**_{**3**}**) : 1,3-1,8 (m,24H) ; 3,10-3,25 (m,8H) ; 4,7 (s,2H) ; 5,0 (t,1H) ; 6,8 (s,1H).**

### PREPARATION XVI

### Acide 3-[[(1,1-diméthyléthoxy)carbonyl]amino]-21-[(1,1-diméthyléthoxy) carbonyl]-1 4-oxa-12,15-dioxo-2,4,11,16,21,25-hexaazahexacos-2-ène dioïque, bis-(1,1-diméthyléthyl) ester.

En opérant de façon analogue à la préparation IV et au départ de 3 g (6,7.10⁻³ moles) du produit obtenu à la préparation XV et de 2,4 g (6,7.10⁻³ moles) d'acide [[(6-aminohexyl)imino]méthylène]bis-carbamique, bis(1,1-diméthyléthyl) ester, on obtient 3,85 g (rendement : 73 %) du produit attendu sous forme d'huile.
**RMN** ^{**1**}**H (CDCl**_{**3**}**, D**_{**2**}**O) : 1,35-1,65 (m,50H) ; 3,10-3,30 (m,12H) ; 4,55 (s,2H) ; 5,2-5,5 (s large,1H) ; 6,4 (s large,1H).**

### PREPARATION XVII

### Acide 3-[[(1,1-diméthyléthoxy)carbonyl]amino]-23-[(1,1-diméthyléthoxy) carbonyl]-16-oxa-14,17-dioxo-2,4,13,18,23,27-hexaazaoctacos-2-ène dioïque, bis-(1,1-diméthyléthyl) ester.

En opérant de façon analogue à la préparation IV et au départ de 1,5 g (3,35.10⁻³ moles) du produit obtenu à la préparation XV et de 1,3 g (3,35.10⁻³ moles) d'acide [[(8-aminooctyl)imino]méthylène]bis-carbamique, bis(1,1-diméthyléthyl) ester, on obtient 1,56 g (rendement : 57 %) du produit attendu sous forme d'huile.
**RMN** ^{**1**}**H (CDCl**_{**3**}**) : 1,40-1,60 (m,54H) ; 3,10-3,40 (m,12H) ; 4,5 (s,2H) ; 5,1-5,4 (s large,1H) ; 6,3 (s large,1H) ; 8,3 (t,1H) ; 11,5 (s large,1H).**

### Exemple 5

### 2-[[[4-[[3-(amino)propyl]amino]butyl]amino]carbonyloxy]-N-[6-[(amino iminométhyl)amino]hexyl]-acétamide, tris-(trifluoroacétate).

En opérant de façon analogue au procédé de l'exemple 1 et au départ de 3,85 g (4,9.10⁻³ moles) du produit obtenu à la préparation XVI, on obtient 2,61 g (rendement : 72 %) du produit attendu sous forme d'huile.
**RMN** ^{**1**}**H (diméthylsulfoxyde-d6) : 1,25-1,55 (m,12H) ; 1,9 (m,2H) ; 2,80-3,10 (m,12H) ; 4,35 (s,2H) ; 5,5-8,6 (m, 12H).**
**RMN** ^{**13**}**C (D**_{**2**}**O) : 23,6 ; 24,5 ; 26,2 ; 26,3 ; 26,7 28,6 ; 29,0 ; 37,3 ; 39,8 ; 40,6 ; 41,9 ; 45,2 ; 48,2 ; 63,7 ; 157,5 ; 158,2 ; 171,4.**

### Exemple 6

### 2-[[[4-[[3-(amino)propyl]amino]butyl]amino]carbonyloxy]-N-[8-[(amino iminométhyl)amino]octyl]-acétamide, tris-(trifluoroacétate).

En opérant de façon analogue au procédé de l'exemple 1 et au départ de 1,56 g (1,9.10⁻³ mole) du produit obtenu à la préparation XVII, on obtient 0,96 g (rendement : 66 %) du produit attendu sous forme d'huile.
**RMN** ^{**1**}**H (diméthylsulfoxyde-d6) : 1,2-1,6 (m,16H) ; 1,9 (m,2H) ; 2,8-3,2 (m,12H) ; 4,35 (s,2H) ; 6,8-8,6 (m,12H).**
**RMN** ^{**13**}**C (D**_{**2**}**O) : 23,6 ; 24,5 ; 26,5 ; 26,6 ; 26,7 ; 28,6 ; 28,9 (2C) ; 29,0 ; 37,9 ; 40,0 ; 40,6 ; 41,9 ; 45,2 ; 48,2; 64,7 ; 158,0 ; 159,0 ; 172,1.**

### PREPARATION XVIII

### Acide 3-[[(1,1-diméthyléthoxy)carbonyl]amino]-13-phénylméthoxy-12-oxo-2,4,11-triazatétradéc-2-ènedioïque, 1-(1,1-diméthyléthyl) 14-éthyl ester.

En opérant de façon analogue à la préparation IV et au départ de 1,3 g (5,46.10⁻³ moles) d'acide 2-phénylméthoxy-propanedioïque, éthyl ester et de 1,95 g (5,46.10⁻³ moles) d'acide [[(6-aminohexyl)imino]méthylène]bis-carbamique, bis-(1,1-diméthyléthyl) ester, on obtient 1,57 g (rendement : 50 %) du produit attendu sous forme d'huile.
**RMN** ^{**1**}**H (CDCl**_{**3**}**) : 1,2-1,8 (m,29H) ; 3,25 (q,2H) ; 3,4 (q,2H)** ; **4,2 (q,2H) ; 4,4 (s,1H) ; 4,5-4,8 (d double,2H) ; 6,7 (s,1H) ; 7,3 (s,5H) ; 8,3 (t,1H) ; 11,5 (s,1H).**

### PREPARATION XIX

### Acide 3-[[(1,1-diméthyléthoxy)carbonyl]amino]-13-phénylméthoxy-12-oxo-2,4,11-triazatétradéc-2-ènedioïque, 1-(1,1-diméthyléthyl) ester.

En opérant de façon analogue à la préparation III et au départ de 1,57 g (2,72.10⁻³ moles) du produit obtenu à la préparation XVIII, on obtient 1,4 g (rendement : 94 %) du produit attendu sous forme d'huile jaunâtre.
**RMN** ^{**1**}**H (CDCl**_{**3**}**) : 1,2-1,8 (m,26H) ; 3,2-3,5 (m,4H) ; 4,4 (s,1H) ; 4,6-5,0 (d double,2H) ; 6,8 (s,1H) ; 7,3 (s,5H) ; 8,3 (t,1H).**

### PREPARATION XX

### Acide 3-[[(1,1-diméthyléthoxy)carbonyl]amino]-20-[(1,1-diméthyléthoxy) carbonyl]-12,14-dioxo-13-phénylméthoxy-2,4,11,15,20,24-hexaaza pentacos-2-ènedioïque, bis-(1,1-diméthyléthyl) ester.

En opérant de façon analogue à la préparation IV et au départ de 1,4 g (2,54.10⁻³ moles) du produit obtenu à la préparation XIX et de 0,88 g (2,54.10⁻³ moles) d'acide 10-amino-6-[(1,1-diméthyléthoxy)carbonyl]-2,6-diazadécanoïque, 1,1-(diméthyléthyl) ester, on obtient 2,1 g (rendement : 94 %) du produit attendu sous forme d'huile.
**RMN** ^{**1**}**H (CDCl**_{**3**}**) : 1,3-1,8 (m,50H) ; 3,1-3,5 (m,12H) ; 4,3 (s,1H) ; 4,8 (s,2H) ; 6,8-7,0 (m,2H) ; 7,3 (s,5H) ; 8,3 (t,1H) ; 11,5 (s,1H).**

### PREPARATION XXI

### Acide 3-[[(1,1-diméthyléthoxy)carbonyl]amino]-20-[(1,1-diméthyléthoxy) carbonyl]-13-hydroxy-12,14-dioxo-2,4,11,15,20,24-hexaazapentacos-2-énedioïque, bis-(1,1-diméthyléthyl) ester.

A une solution de 1,27 g (1,45.10⁻³ mole) du produit obtenu à la préparation XX dans 120 ml d'éthanol, on ajoute 0,1 g de charbon palladié à 10 %. On agite à température ambiante et sous atmosphère d'hydrogène à pression atmosphérique pendant 2 heures. Le catalyseur est ensuite éliminé par filtration et la phase organique est évaporée. On obtient ainsi 1 g (rendement : 88 %) de résidu huileux qui est utilisé sans autre purification pour la préparation du produit de l'exemple 7.
**RMN** ^{**1**}**H (CDCI**_{**3**}**) : 1,2-1,8 (m,50H) ; 3,1-3,6 (m,12H) ; 4,3 (s,1H)** ; **4,6-5,3 (s large,3H) ; 8,3 (s,1H) ; 11,5 (s,1H).**

### Exemple 7

### N-14-[[3-(amino)propyl]amino]butyl]-2-hydroxy-N'-[6-[(aminoiminométhyl) amino]hexyl]-propanediamide, tris-(trifluoroacétate).

En opérant de façon analogue au procédé de l'exemple 1 et au départ de 1 g (1,27.10⁻³ mole) du produit obtenu à la préparation XXI, on obtient 0,6 g (rendement : 65 %) du produit attendu sous forme d'huile.
**RMN** ^{**1**}**H (diméthylsulfoxyde-d6) : 1,2-1,6 (m,12H) ; 1,85 (m,2H) ; 2,7-3,2 (m,12H) ; 4,3 (s,1H) ; 6,8-8,6 (m,12H).**
**RMN** ^{**13**}**C (D**_{**2**}**O) : 23,5 ; 24,4 ; 26,0 ; 26,1; 26,2 : 28,4 ; 28,8 ; 37,2** ; **39,1 ; 39,9 ; 41,7 ; 45,1 ; 48,0 : 73,0; 154,8; 171,1 ; 171,4.**

### PREPARATION XXII

### Acide 3-[[(1,1-diméthyléthoxy)carbonyl]amino]-12-oxo-2,4,11,14-tétra azapentadéc-2-ènedioïque, 1-(1,1-diméthyléthyl) 15-phénylméthyl ester.

A une solution refroidie à -30°C de 3 g (14.10⁻³ moles) de carbobenzyloxyglycine et de 2,8 g (28.10⁻³ moles) de N-méthyl morpholine dans 50 ml de tétrahydrofuranne, on ajoute goutte à goutte 1,6 g (14.10⁻³ moles) de chloroformiate d'isobutyle en solution dans 5 ml de tétrahydrofuranne. On agite le milieu réactionnel pendant 0,5 heure et on additionne 5,4 g (14.10⁻³ moles) d'acide [[(6-aminohexyl) imino]méthylène]bis-carbamique, bis-(1,1-diméthyléthyl) ester en solution dans 20 ml de tétrahydrofuranne. On agite encore 2 heures à - 30°C puis 24 heures à température ambiante. Après filtration du milieu réactionnel et évaporation du filtrat sous pression réduite, le résidu obtenu est purifié par chromatographie sur silice en éluant avec un mélange acétate d'éthyle/méthylcyclohexane 1/1 (v/v). On obtient ainsi 7,16 g (rendement : 91 %) du produit attendu sous forme d'huile.
**RMN** ^{**1**}**H (CDCl**_{**3**}**) : 1,3-1,7 (m,26H) ; 3,2 (q,2H) ; 3,4 (q,2H) ; 3,8 (d,2H) ; 5,15 (s,2H) ; 5,5 (s large,1H) ; 6,0 (s large,1H) ; 7,3 (s,5H).**

### PREPARATION XXIII

### Acide 13-amino-3-[[(1,1-diméthyléthoxy)carbonyl]amino]-12-oxo-2,4,11-triazatridéc-2-ènoïque, 1,1-diméthyléthyl ester.

En opérant de manière analogue à la préparation XXI et au départ de 7,1 g (13.10⁻³ moles) du produit obtenu à la préparation XXII, on obtient 5,3 g (rendement : 98 %) du produit attendu sous forme d'huile.
**RMN** ^{**1**}**H (CDCl**_{**3**}**) : 1,3-1,6 (m,28H) ; 3,25-3,45 (m,6H) ; 7,3 (s,1H) ; 8,3 (t,1H) ; 11,5(s,1H).**

### PREPARATION XXIV

### Acide 3-[[(1,1-diméthyléthoxy)carbonyl]amino]-21-[(1,1-diméthyléthoxy) carbonyl]-12,15-dioxo-2,4,11,14,16,21,25-heptaazahexacos-2-ène dioïque, bis-(1,1-diméthyléthyl) ester.

A une solution de 5,3 g (12.10⁻³ moles) du produit obtenu à la préparation XXIII dans 50 ml de tétrahydrofuranne anhydre, on ajoute 4,3 g (13.10⁻³ moles) de bis(4-nitrophényl)carbonate par petites fractions. On agite le milieu réactionnel pendant 1 heure à température ambiante et on additionne goutte à goutte 4,5 g (13.10⁻³ moles) d'acide 10-amino-6-[(1,1-diméthyléthoxy)carbonyl]-2,6-diazadécanoïque, 1,1-(diméthyléthyl) ester en solution dans 50 ml de tétrahydrofuranne anhydre. On agite à nouveau 24 heures à température ambiante et on évapore le solvant sous pression réduite. Le résidu obtenu est purifié par chromatographie sur silice en éluant à l'acétate d'éthyle. On obtient ainsi 6,01 g (rendement : 64 %) du produit attendu sous forme d'huile.
**RMN** ^{**1**}**H (CDCl**_{**3**}**) : 1,3-1,7 (m,50H) ; 3,1-3,35 (m,12H) ; 3,8 (d,2H) ; 4,8 et 5,8 (s large,3H) ; 6,9 (t,1H) ; 8,3 (t,1H) ; 11,5 (s,1H).**

### Exemple 8

### N-[4-[[3-(amino)propyl]amino]butyl]-N'-[[[[6-[(aminoiminométhyl)amino] hexyl]amino]carbonyl]méthyl]-urée, tris-(trifluoroacétate).

En opérant de façon analogue au procédé de l'exemple 1 et au départ de 6 g (7,6.10⁻³ moles) du produit obtenu à la préparation XXIV, on obtient 4,75 g (rendement : 86 %) du produit attendu sous forme d'huile.
**RMN** ^{**1**}**H (diméthylsulfoxyde-d6) : 1,2-1,65 (m,12H) ; 1,9 (m,2H) ; 2,9-3,15 (m,12H) ; 3,6 (d,2H) ; 6,1 (t,1H) ; 6,3 (t,1H) ; 6,8-9 (m,11H).**
**RMN** ^{**13**}**C (diméthylsulfoxyde-d6) : 22,8 ; 23,7 ;25,6 ; 25,8 ; 27,0 ; 28,3 ; 28,9 ; 36,1; 38,3 ; 38,5 ; 40,5 ; 42,7 ; 43,7 ; 46,5 ; 156,7 ; 157,9 ; 169,6.**

### PREPARATION XXV

### Acide 3-[[(1,1-diméthyléthoxy)carbonyl]amino]-14-oxo-2,4,13,16-tétra azaheptadéc-2-ènedioïque, 1 -(1,1 -diméthyléthyl) 17-phénylméthyl ester.

En opérant de façon analogue à la préparation IV et au départ de 1,35 g (6,47.10⁻³ moles) de carbobenzyloxyglycine et de 2 g (5,18.10⁻³ moles) d'acide [[(8-aminooctyl)imino]méthylène]bis-carbamique, bis-(1,1-diméthyléthyl) ester, on obtient 2,33 g (rendement : 74 %) du produit attendu sous forme d'huile.
**RMN** ^{**1**}**H (CDCl**_{**3**}**) : 1,25-1,70 (m,30H) ; 3,25 (q,2H) ; 3,4 (q,2H) ; 3,8 (d,2H) ; 5,15 (s,2H) ; 5,5 (s large,1H) ; 6,0 (s large,1H) ; 7,3 (s,5H) ; 8,3 (t,1H) ; 11,5 (s,1H).**

### PREPARATION XXVI

### Acide 15-amino-3-[[(1,1-diméthyléthoxy)carbonyl]amino]-14-oxo-2,4,13-triazapentadéc-2-ènoïque, 1,1-diméthyléthyl ester.

En opérant de façon analogue à la préparation XXI et au départ de 2,33 g (4,04.10⁻³ moles) du produit obtenu à la préparation XXV, on obtient 2,16 g (rendement : 100 %) du produit attendu sous forme d'huile.
**RMN** ^{**1**}**H (CDCl**_{**3**}**)** : **1,3-1,8 (m,32H) ; 3,25-3,45 (m,6H) ; 7,3 (s,1H) ; 8,3 (t,1H) ; 11,5 (s,1H).**

### PREPARATION XXVII

### Acide 3-[[(1,1-diméthyléthoxy)carbonyl]amino]-23-[(1,1-diméthyléthoxy) carbonyl]-14,17-dioxo-2,4,13,16,18,23,27-heptaazaoctacos-2-ène dioïque, bis(1,1-diméthyléthyl) ester.

En opérant de manière analogue à la préparation XXIV et au départ de 1,79 g (4,04.10⁻³ moles) du produit obtenu à la préparation XXVI, on obtient 1,5 g (rendement 45 %) du produit attendu sous forme d'huile.
**RMN** ^{**1**}**H (CDCl**_{**3**}**) : 1,3-1,7 (m,54H) ; 3,1-3,4 (m,12H) ; 3,8 (d,2H) ; 4,8, 5,2 et 5,7 (s large,2H) ; 6,0 et 6,7 (s large,1H) ; 8,3 (t,1H) ; 11,5 (s,1H).**

### Exemple 9

### N-[4-[[3-(amino)propyl]amino]butyl]-N'-[[[[8-[(aminoiminométhyl)amino] octyl]amino]carbonyl]méthyl]-urée, tris-(trifluoroacétate).

En opérant de façon analogue au procédé de l'exemple 1 et au départ de 1,5 g (1,84.10⁻³ mole) du produit obtenu à la préparation XXVII, on obtient 1,14 g (rendement : 82 %) du produit attendu sous forme d'huile.
**RMN**^{**1**}**H (diméthylsulfoxyde-d6) : 1,25-1,55 (m,16H) ; 1,9 (m,2H) ; 2,9-3,10 (m,12H) 3,6 (s,2H) ; 6,0-6,3 (s large,2H) ; 6,8-8,6 (m, 11H).**
**RMN** ^{**13**}**H (diméthylsulfoxyde-d6) : 22,9 ; 23,8 ; 26,0 ; 26,3 ; 27,1 ; 28,4 ; 28,5 ; 28,6 ; 29,1 ; 36,2 ; 38,4 ; 38,7 ; 40,7 ; 42,7 ; 43,8** ; **46,6 ; 156,7 ; 158,0 ; 169,6.**

### PREPARATION XXVIII

### Acide 3-[[(1,1-diméthyléthoxylcarbonyl]amino]-12-oxo-2,4,11.triazatri déc-2-énedioïque, 1-(1,1-diméthyléthyl) 13 éthyl ester.

A une solution de 1,6 g (4,5.10⁻³ moles) d'acide [[(6-aminohexyl) imino]méthyléne]bis-carbamique, bis(1,1-diméthyléthyl) ester et 0,6 g (6.10⁻³ moles) de triéthylamine dans 10 ml de dichlorométhane anhydre, on ajoute, goutte à goutte, 0,67 g 15.10⁻³ moles) du chlorure d'acide de l'oxalate d'éthyle en solution dans 5 ml de dichlorométhane. On agite 1 heure à température ambiante, puis on évapore le solvant sous pression réduite et le résidu obtenu est chromatographié sur silice en éluant avec un mélange hexane/acétate d'éthyle 2/1 (v/v). On obtient ainsi 1,68 g (rendement : 82 %) du produit attendu sous forme d'huile.
**RMN** ^{**1**}**H (CDCl**_{**3**}**) : 1,3-1,7 (m,29H) ; 3,3-3,4 (m,4H) ; 4,4 (q,2H) ; 7,1 (t,1H) ; 8,3 (t,1H) ; 11,5 (s.1H).**

### PREPARATION XXIX

### Acide 3-[[(1,1-diméthyléthoxy)carbonyl]amine]-12-oxo-2,4,11-triazatri déc-2-ènedioïque, 1-(1,1-diméthyléthyl) ester.

En procédant de façon analogue à la préparation III et au départ de 1,67 g (3,7.10⁻³ moles) du produit obtenu à la préparation XXVIII, on obtient 1,2 g (rendement : 75 %) du produit attendu sous forme d'huile.
**RMN** ^{**1**}**H (CDCl**_{**3**}**) : 1,25-1,7 (m,26H) ; 3,3-3,5 (m,4H) ; 7,4 (t,1H) ; 8,5 (t,1H) ; 11,5 (s large,1H).**

### PREPARATION XXX

### Acide 3-[[(1,1-diméthyléthoxy)carbonyl]amino]-19-[(1,1-diméthyléthoxy) carbonyl]-12,13-dioxo-2,4,11,14,19,23-hexaazatétracos-2-ènedioïque, bis-(1,1-diméthyléthyl) ester.

En opérant de façon analogue à la préparation XXII et au départ de 1,4 g (3,5.10⁻³ moles) du produit obtenu à la préparation XXIX et de 1,05 g (3.10⁻³ moles) d'acide **1**0-amino-6-[(1,1-diméthyléthoxy)carbonyl]-2,6-diazadécanoïque, 1,1-(diméthyléthyl) ester, on obtient 1,2 g (rendement : 53 %) du produit attendu sous forme d'huile.
**RMN** ^{**1**}**H (CDCl**_{**3**}**) : 1,3-1,7 (m,50H) ; 3,1-3,4 (m, 12H) ; 4,8 et 5,3 (s large,1H) ; 7,5 (m,2H) ; 8,3 (t,1H) ; 11,5 (s,1H).**

### Exemple 10

### N-[4-[[3-(amino)propyl]amino]butyl]-N'-[6-[(aminoiminométhyl)amino] hexyl]-éthanediamide, tris-(trifluoroacétate).

En opérant de façon analogue au procédé de l'exemple 1 et au départ de 1,2 g (1,6.10⁻³ mole) du produit obtenu à la préparation XXX, on obtient 1,05 g (rendement : 95 %) du produit attendu sous forme d'huile.
**RMN** ^{**1**}**H (diméthylsulfoxyde-d6) : 1,3-1,65 (m,12H) ; 1,9 (m,2H) ; 2,8-3,3** **(m,12H) 6,8-9,9 (m,12H).**
**RMN** ^{**13**}**C (diméthylsulfoxyde-d6,D**₂**O) : 23,9 ; 24,7 ; 26,3 ; 26,4 ; 26,5 ; 28,7 ; 29,0 ; 37,5 ; 39,7 ; 40,4 ; 42,0 ; 45,4 ; 48,1 ; 157,7 ; 161,8 ; 162,1.**

### PREPARATION XXXI

### Acide 6-[(1,1-diméthyléthoxy)carbonyl]-12-oxo-2,6,11-triazatridécane dioïque, 1-(1,1-diméthyléthyl) 13-éthyl ester.

En opérant de façon analogue à la préparation XXVIII et au départ de 2 g (5,8.10⁻³ moles) d'acide 10-amino-6-[(1,1-diméthyléthoxy)carbonyl]-2,6-diazadécanoïque, 1,1-(diméthyléthyl) ester et de 1,03 g (7,54.10⁻³ moles) du chlorure d'acide de l'oxalate d'éthyle, on obtient 2,31 g (rendement : 89 %) du produit attendu sous forme d'huile.
**RMN** ^{**1**}**H (CDCl**_{**3**}**) : 1,25-1,65 (m,27H) ; 3,1-3,35 (m,8H) ; 4,35 (q,2H) ; 4,75 et 5,25 (s large,1H) ; 7,2 (s,1H).**

### PREPARATION XXXII

### Acide 6-[(1,1-diméthyléthoxy)carbonyl]-12-oxo-2,6,11-triazatridécane dioïque, 1-(1,1-diméthyléthyl) ester.

En opérant de façon analogue à la préparation III et au départ de 2,31 g (5,19.10⁻³ moles) du produit obtenu à la préparation XXXI, on obtient 2,16 g (rendement : 100 %) du produit attendu sous forme d'huile.
**RMN** ^{**1**}**H (CDCl**_{**3**}**) : 1,25-1,70 (m,24H) ; 3,1-3,4 (m,8H) ; 4,8 - 5,25 (s double,1H) ; 7,5 (s large,1H).**

### PREPARATION XXXIII

### Acide 3-[[(1,1-diméthyléthoxy)carbonyl]amino]-21-[(1,1-diméthyléthoxy) carbonyl]-14,15-dioxo-2,4,13,16,21,25-hexaazahexacos-2-ènedioïque, bis-(1,1-diméthyléthyl) ester.

En opérant de façon analogue à la préparation IV et au départ de 1 g (2,4.10⁻³ moles) du produit obtenu à la préparation XXXII et de 0,92 g (2,4.10⁻³ moles) d'acide[[(8-aminooctyl)imino]méthylène]bis-carbamique, bis-(1,1-diméthyléthyl) ester, on obtient 0,9 g (rendement : 48 %) du produit attendu sous forme d'huile.
**RMN** ^{**1**}**H (CDCl**_{**3**}**) : 1,3 (t,3H) ; 1,4-1,7 (m,24H) ; 3,0-3,4 (m,8H) ; 4,25 (q,2H) ; 4,4 (s,1H) ; 4,5-4,7 (2d,2H) ; 5,0 (s,1H) ; 6,7 (s,1H) ; 7,3 (m,5H).**

### Exemple 11

### N-[4-[[3-(amino)propyl]amino]butyl]-N'-[8-[(aminoiminométhyl)amino] octyl]-éthanediamide, tris-(trifluoroacétate).

En opérant de façon analogue au procédé de l'exemple 1 et au départ de 0,9 g (1,15.10⁻³ mole) du produit obtenu à la préparation XXXIII, on obtient 0,15 g (rendement : 18 %) du produit attendu sous forme d'huile.
**RMN** ^{**1**}**H (diméthylsulfoxyde-d6) : 1,25-1,50 (m,16H) ; 1,9 (m,2H) ; 2,9-3,15 (m,12H) ; 6,9-8,8 (m, 12H).**
**RMN** ^{**13**}**C (D**_{**2**}**O) : 23,8 ; 24,5 ; 26,1 ; 26,5 ; 26,7 ; 28,6 ; 28,92 ; 28,95 ; 29,0 ; 37,3 ; 39,5 ; 40,4 ; 42,0 ; 45,2 ; 48,1.**

### PREPARATION XXXIV

### Acide 6-[(1,1-diméthyléthoxy)carbonyl]-12-oxo-13-phénylméthoxy-2,6, 11-triazatétradécanedioïque, 1-(1,1-diméthyléthyl) 14-éthyl ester.

En opérant de façon analogue à la préparation IV et au départ de 3 g (12,6.10⁻³ moles) d'acide 2-(phénylméthoxy)propanedioïque, éthyl ester et de 4,4 g (12,6.10⁻³ moles) d'acide 10-amino-6-[(1,1-diméthyl éthoxy)carbonyl]-2,6-diazadécanedioïque, 1,1-diméthyléthyl ester, on obtient, après purification par chromatographie sur silice (éluant **:** méthylcyclohexane 8/acétate d'éthyle 2), le produit attendu avec un rendement de 35 %.
**RMN** ^{**1**}**H (CDCl**_{**3**}**) : 1,3 (t,3H) ; 1,4-1,7 (m,24H) ; 3,0-3,4 (m,8H) ; 4,25 (q ,2H) ; 4,4 (s,1H) ; 4,5-4,7 (2d,2H) ; 5,0 (s,1H) ; 6,7 (s,1H) ; 7,3 (m,5H).**

### PREPARATION XXXV

### Acide 6-[(1,1-diméthyléthoxy)carbonyl]-12-oxo-13-phénylméthoxy-2,6, 11-triazatétradécanedioïque, 1-(1,1-diméthyléthyl) ester.

On dissout 2,46 g (4,3.10⁻³ moles) du produit obtenu à la préparation XXXIV dans un mélange éthanol/eau (1/1 en volume) et on ajoute 258 mg de soude. Le milieu réactionnel est agité à température ambiante pendant 24 heures puis on ajoute 10 ml d'eau et 20 ml de chloroforme et on acidifie jusqu'à pH2 avec de l'acide chlorhydrique N. On extrait avec du chloroforme et, après séchage sur sulfate de magnésium, la phase organique est concentrée sous pression réduite. On obtient ainsi le produit attendu avec un rendement de 76 %.
**RMN** ^{**1**}**H (CDCl**_{**3**}**) : 1,4-1,7 (m,24H) ; 3,0-3,3 (m,8H) ; 4,4-4,5 (m,1H) ; 4,7-5,1 (2d,2H) ; 5,0 (s,1H) ; 6,65 (s,1H) ; 7,4 (m,5H).**

### PREPARATION XXXVI

### Acide 3-[[(1,1-diméthyléthoxy)carbonyl]amino]-22-[(1,1-diméthyléthoxy) carbonyl]-14,16-dioxo-15-phénylméthoxy-2,4,13,17,22,26-hexaaza heptacos-2-ènedioïque,bis-(1,1-diméthyléthyl) ester.

En opérant de façon analogue à la préparation IV et au départ de 1,67 g (3,11.10⁻³ moles) du produit de la préparation XXXV et de 1,20 g (3,11.10⁻³ moles) d'acide [[(8-aminooctyl)imino]méthylène]bis-carba mique, bis-(1,1-diméthyléthyl) ester, on obtient, après purification par chromatographie sur silice (éluant : méthylcyclohexane 7/acétate d'éthyle 3), le produit attendu avec un rendement de 59 %, sous forme d'huile.
**RMN** ^{**1**}**H (CDCl**_{**3**}**) : 1,2-1,8 (m,54H) ; 3,0-3,5 (m,12H) ; 4,3 (s,1H) ; 4,8 (m,2H) ; 5,1 (s,1H) ; 6,9-7,1 (m,2H) ; 7,4 (m,5H) ; 8,3 (t,1H) ; 11,5 (s,1H).**

### PREPARATION XXXVII

### Acide 3-[[(1,1-diméthyléthoxy)carbonyl]amino]-22-[(1,1-diméthyléthoxy) carbonyl]-14,16-dioxo-15-hydroxy-2,4,13,17,22,26-hexaazaheptacos-2-ènedioïque,bis-(1,1-diméthyléthyl) ester.

En opérant de façon analogue à la préparation XXI et au départ de 1,64 g du produit obtenu à la préparation XXXVI, on obtient le produit attendu avec un rendement de 55 %.
**RMN** ^{**1**}**H (CDCl**_{**3**}**) : 1,3-1,7 (m,54H) ; 3,0-3,4 (m,12H) ; 4,4 (s,1H) ; 5,0 (s,1H) ; 7,2 (s,1H) ; 7,35 (s,1H) ; 8,3 (t,1H) ; 11,5(s,1H).**

### Exemple 12

### N-[4-[[3-(amino)propyl]amino]butyl]-2-hydroxy-N'-[8-[(aminoiminométhyl) amino]octyl]-propanediamide, tris-(trifluoroacétate).

En opérant de façon analogue au procédé de l'exemple 1, au départ de 0,81 g du produit obtenu à la préparation XXXVII, on obtient, après purification par chromatographie MPLC sur silice greffée RP18 (éluant : eau 7,5/acétonitrile 1,5/acide trifluoroacétique 1) le produit attendu avec un rendement de 67 %, sous forme d'huile.
**RMN** ^{**1**}**H (DMSO-d6) : 1,1-1,7 (m,16H) ; 1,9 (m,2H) ; 2,8-3,2 (m,12H) ; 4,3 (s,1H) ; 6,9-8,7 (m,12H).**
**RMN** ^{**13**}**C (DMSO-d6) : 14,8 ; 20,9 ; 22,2 ; 23,1; 25,3 ; 25,9 ; 25,5 ; 28,7 ; 28,9 ; 36,8 ; 38,1 ; 43,8 ; 46,2 ; 60,1 ; 71,8 ; 157,3 ; 166,2 ; 166,5.**

### PREPARATION XXXVIII

### Acide 6-[(1,1-diméthyléthoxy)carbonyl]-13-fluoro-12-oxo-2,6,11-triaza tétradécanedioïque, 1-(1,1-diméthyléthyl) 14-éthyl ester.

On prépare une solution de 1,7 g (11.10⁻³ moles) d'acide 2-fluoro-propanedioïque, éthyl ester et de 2,22 g (22.10⁻³ moles) de N-méthyl-morpholine dans 50 ml de tétrahydrofurane (THF) et 5 ml de diméthylformamide anhydres. On refroidit ensuite le mélange à -20°C, puis on ajoute 1,6 ml (12.10⁻³ moles) de chloroformiate d'isobutyle en solution dans 5 ml de THF. On agite 30 mn à -20°C puis on ajoute 3,9 g (11.10⁻³ moles) d'acide 10-amino-6-[(1,1-diméthyléthoxy)carbonyl]-2,6-di azadécanoïque, 1,1-diméthyléthyl ester, en solution dans 30 ml de THF. On maintient sous agitation pendant 2 heures à -20°C puis pendant 12 heures à température ambiante. On filtre le milieu réactionnel et concentre le filtrat sous pression réduite. Le résidu obtenu est purifié par chromatographie sur silice (éluant : méthylcyclohexane 7/acétate d'éthyle 3). On obtient le produit attendu avec un rendement de 34 % sous forme d'huile.
**RMN** ^{**1**}**H (CDCl**_{**3**}**) : 1,34 (t,3H) ; 1,4-1,7 (m,24H) ; 3,0-3,4 (m,8H) ; 4,3 (m,2H) ; 5,0 (s,1H) ; 5,25 (d,1H) ; 6,55 (s,1H).**

### PREPARATION XXXIX

### Acide 6-[(1,1-diméthyléthoxy)carbonyl]-13-fluoro-12-oxo-2,6,11-triaza tétradécanedioïque, 1-(1,1-diméthyléthyl) ester.

On prépare une solution de 1,8 g (3,8.10⁻³ moles) du produit obtenu à la préparation XXXVIII dans 6 ml de soude aqueuse N et 20 ml de diméthoxyéthane. Après une heure d'agitation à température ambiante on ajoute 10 ml d'eau et 20 ml de dichlorométhane, on acidifie jusqu'à pH2 avec de l'acide chlorhydrique N. On extrait avec deux fois 20 ml de dichlorométhane, sèche la phase organique sur sulfate de magnésium et concentre sous pression réduite. On obtient ainsi 1,55 g du produit attendu sous forme d'huile (rendement : 91 %)
**RMN** ^{**1**}**H (CDCl**_{**3**}**) : 1,4-1,7 (m,24H) ; 3,0-3,35 (m,8H) ; 5,0 (d,1H) ; 5,3 (d,1H) ; 6,5 (s,1H).**

### PREPARATION XL

### Acide 3-[[(1,1-diméthyléthoxy)carbonyl]amino]-20-[(1,1-diméthyléthoxy) carbonyl]-12,14-dioxo-13-fluoro-2,4,11,15,20,24-hexaazapentacos-2-ènedioïque, bis-(1,1-diméthyléthyl) ester.

A une solution de 1 g (2,2.10⁻³ moles) obtenu suivant la préparation XXXIX et de 4,5 g (4,4.10⁻³ moles) de N-méthylmorpholine dans 30 ml de THF anhydre, refroidie à -20°C, on ajoute goutte à goutte 0,29 ml (2,2.10⁻³ moles) de chloroformiate d'isobutyle en solution dans 5 ml de THF. On agite 30 mn à -20°C, puis on ajoute 0,876 g (2,2.10⁻³ moles) d'acide [[(6-aminohexyl)imino]méthylène]bis-carbamique, bis-(1,1-diméthyléthyl) ester et 0,34 ml (2,2.10⁻³ moles) de triéthylamine en solution dans 5 ml de THF. On maintient la température à -20°C pendant deux heures, puis on revient à température ambiante et on continue à agiter pendant 12 heures. Le milieu réactionnel est filtré puis concentré sous pression réduite. Le résidu est ensuite purifié par chromatographie sur silice (éluant : acétate d'éthyle 7/cyclohexane 3) et on obtient 1,43 g du produit attendu sous forme d'huile (rendement 81 %).
**RMN** ^{**1**}**H (CDCl**_{**3**}**) : 1,3-1,7 (m,50H) ; 3,0-3,45 (m,12H) ; 5,0 (s,1H) ; 5,2 (d,1H) ; 6,8-7,1 (m,2H) ; 8,3 (t,1H) ; 11,5 (s,1H).**

### exemple 13

### N-[4-[[3-(amino)propyl]amino]butyl]-N'-[6-[(aminoiminométhyl)amino] hexyl]-2-fluoro-propanediamide, tris-(trifluoroacétate).

En opérant de façon analogue au procédé de l'exemple 1 et au départ de 0,4 g (0,5.10⁻³ mole) du produit obtenu à la préparation XL on obtient, après purification par chromatographie MPLC sur silice greffée RP18 (éluant : eau 7,5/acétonitrile 2/ acide triflutoacétique 0,5), 263 mg du produit attendu sous forme d'huile (rendement 71 %).
**RMN** ^{**1**}**H (DMSO-d**^{**6**}**) : 1,25-1,55 (m,12H) ; 1,9 (m,2H) ; 2,7-3,15 (m,12H) ; 5,2 (d,1H) ; 6,9-8,7 (m,12H).**
**RMN** ^{**13**}**C (D**_{**2**}**O/Dioxane-d**^{**8**}**) : 23,65 ; 24,53 ; 26,17 ; 26,25 ; 28,55 ; 28,79 ; 37,34 ; 39,32 ; 40,08 ; 41,86 ; 45,22 ; 48,09 ; 87,00 ; 89,66 ; 157,0 ; 166,2 ; 166,7.**

### PREPARATION XLI

### Acide 3-[[(1,1-diméthyléthoxy)carbonyl]amino]-22-[(1,1-diméthyléthoxy) carbonyl]-15-fluoro-14,16-dioxo-2,4,13,17,22,26-hexaazaheptacos-2-ènedioïque, bis-(1,1-diméthyléthyl) ester.

En opérant de manière analogue à la préparation XXII et au départ de 0,460 g (1,02.10⁻³ mole) du produit obtenu selon la préparation XXXIX et de 0,395 g (1,02.10⁻³ moles) d'acide [[(8-aminooctyl) imino]méthylène]bis-carbamique, bis-(1,1-diméthyléthyl) ester, on obtient après purification par chromatographie sur silice (éluant : acétate d'éthyle 6/cyclohexane 4), 0,513 g du produit attendu sous forme d'huile (Rendement 61 %)
**RMN** ^{**1**}**H (CDCl**_{**3**}**) : 1,2-1,7 (m,54H) ; 3,0-3,4 (m,12H) ; 5,0 (s,1H) ; 5,2 (d,1H) ; 6,8-7,1 (m,2H) ; 8,3 (t,1H) ; 11,5 (s,1H).**

### Exemple 14

### N-[4-[[3-(amino)propyl]amino]butyl]-2-fluoro-N'-[8-[(aminoiminométhyl) amino]octyl]-propanediamide, tris-(trifluoroacétate).

En opérant de façon analogue à la préparation XXII et au départ de 0,460 g (1,02.10⁻³ moles) du produit obtenu selon la préparation XLI, on obtient après purification par chromatographie MPLC sur silice greffée RP18, le produit attendu sous forme d'huile.
**RMN** ^{**1**}**H (DMSO.d6) : 1,25-1,50 (m,1H) ; 1,84 (m,2H) ; 2,7-3,15 (m,12H) ; 5,20 (d,1H) ; 6,9-8,7 (m,12H).**
**RMN** ^{**13**}**C (D**_{**2**}**O/Dioxane-d8): 23,66 ; 24,55 ; 26,18 ; 26,28 ; 26,57 ; 27,48 ; 28,86 ; 37,35 ; 39,31; 40,21; 40,33 ; 45,23 ; 48,10 ; 87,26-89,86 ; 157,0 ; 166,2 ; 166,7**.

### Exemple 15

### N-[4-[[3-(amino)prepyl]amine]butyl]-N'-[6-[(amineiminométhyl)amine] hexyl]-propanediamide, tris-chlorhydrate

### Méthode A :

On dissout 1 g (1,4.10⁻³ moles) du produit obtenu à l'exemple 1, dans un mélange de 7 ml d'acide chlorhydrique 10 M et 50 ml d'eau distillée puis on lyophilise la solution obtenue. Cette opération est répétée deux fois. Le résidu de lyophilisation est repris dans un mélange de 9 ml d'éthanol et 1 ml de méthanol. Les cristaux obtenus après 24 heures à température ambiante sont filtrés, rincés à l'éther isopropylique et séchés sous vide. On obtient ainsi le produit attendu avec un rendement de 68 %, sous forme de cristaux blancs.
F = 130°C

### Méthode B:

On dissout 2,5 g (3,28.10⁻³ moles) du produit obtenu selon la préparation IV dans 25 ml de méthanol saturé par du chlorure d'hydrogène. Après une nuit d'agitation à température ambiante, la solution est concentrée sous pression réduite et le résidu est repris en solution dans 10 ml d'eau puis lyophilisé. Le composé ainsi obtenu est recristallisé dans un mélange éthanol-méthanol (9/1). On obtient ainsi le produit cristallisé avec un rendement de 52 %.

### Exemple 16

### 2-[[[4-[[3-(amino)propyl]amino]butyl]amino]carbonyloxy]-N-[6-[(amino iminométhyl)amino]hexyl]-acétamide, tris-chlorhydrate.

En opérant de façon analogue au procédé de l'exemple 15 (méthode B) et au départ du produit obtenu selon la préparation XVI, on obtient le produit attendu sous forme de cristaux avec un rendement de 58 %.
**F = 148° C**

L'activité immunosuppressive des produits selon l'invention a été mise en évidence à l'aide d'un test de réaction du greffon contre l'hôte. Des souris mâles B6D2F1 (hybrides de première génération C57B1/6 x DBA/2) sont immunodéprimées par une injection intrapéritonéale (i.p.) de cyclophosphamide. Trois jours après (jour 0 de l'expérience : J0), elles reçoivent, par voie intraveineuse, 4 x 10⁷ splénocytes de souris C57B1/6. Les animaux sont ensuite répartis par lot de 8 au minimum et reçoivent un traitement journalier de J1 à J5 et de J7 à J10 par voie i.p. Le groupe contrôle reçoit le véhicule seul. La mortalité est suivie jusqu'à J60. Les résultats, exprimés par la valeur moyenne de la survie en jours à la dose indiquée, sont regroupés dans le tableau I où les valeurs données sont significatives selon le test de Logrank (probabilité inférieure ou égale à 5 %). A fin de comparaison, on a également indique dans le tableau I les valeurs obtenues avec les produits connus de l'art antérieur : la 15-déoxyspergualine (DSG), la cyclosporine A qui est actuellement l'immunosuppresseur de référence utilisé en thérapeutique, et le produit de l'exemple 1 décrit dans EP-A-0 105 193. Il ressort de cette comparaison que les produits selon l'invention sont jusqu'à 250 fois plus actifs que les produits connus de l'art antérieur. Notamment, le produit de l'exemple 1 selon l'invention présente une activité significative dès 0,1 mg/kg alors que le produit de comparaison de l'exemple 1 de EP-A-0 105 193 ne présente une activité significative qu'à partir de 1 mg/kg, la 15-déoxyspergualine qu'à partir de 0,3 mg/kg et la cyclosporine A qu'à partir de 25 mg/kg.

De plus, les composés selon l'invention présentent une stabilité en solution nettement plus grande que les produits connus de l'art antérieur, notamment la 1 5-déoxyspergualine.

Les produits selon l'invention sont utiles en thérapeutique en tant qu'agents immunosuppresseurs curatifs ou préventifs, notamment dans la prévention du rejet d'organes allogéniques ou xénogéniques vascularisés ou non, de la réaction du greffon contre l'hôte suite à une greffe vascularisée ou non, dans le traitement des maladies autoimmunes génétiquement définies ou acquises, des maladies inflammatoires chroniques ainsi que dans toutes les pathologies où un désordre immunitaire apparaît être la cause ou le facteur responsable du maintien d'un état clinique dégradé.

Les produits selon l'invention peuvent également être administrés en complément de drogues anticancéreuses cytotoxiques afin d'en limiter les effets secondaires et en complément de l'administration de produits issus des biotechnologies, notamment les cytokines recombinantes, les anticorps mono- et polyclonaux afin de diminuer l'apparition des anticorps protecteurs produits par le patient.

Les produits selon l'invention peuvent être utilisés en traitement curatif de parasitoses, en particulier dans le cas du paludisme.

Les produits selon l'invention peuvent être administrés par voie orale, injectable, notamment intra-musculaire ou intra-veineuse, topique, notamment sous forme de crème pour application locale, de gouttes occulaires, par voie transdermique, par suppositoire ou par inhalation.

**TABLEAU I**

| Exemples * | n | A | Activités | |
|---|---|---|---|---|
| | | | Dose (mg/kg) | Survie (jours) |
| 1 | 6 | CH₂ | 0,1 | 27,6 |
| | | | 0,3 | 48,5 |
| 2 | 8 | CH₂ | 3 | 47,0 |
| 3 | 6 | CH(OCH₃) | 3 | 55,3 |
| 4 | 8 | CH(OCH₃) | 3 | 27,6 |
| 5 | 6 | CH₂O | 3 | 57,9 |
| 7 | 6 | CH(OH) | 3 | 53,0 |
| 8 | 6 | CH₂NH | 0,3 | 39,8 |
| 10 | 6 | simple liaison | 1 | 52,9 |
| 11 | 8 | simple liaison | 3 | 59,4 |
| 12 | 8 | CH(OH) | 1 | 55,6 |
| 13 | 6 | CHF | 3 | 60,0 |
| 15-déoxyspergualine | | | 1 | 43,1 |
| 15-déoxyspergualine | | | 0,3 | 32,1 |
| CYCLOSPORINE A | | | 25 | 36,0 |
| Ex. 1 de EP-A-O 105 193 | | | 1 | 32,0 |

| | | | | |
|---|---|---|---|---|
| * sous forme de tris-(trifluoroacétate) pour les produits selon l'invention. | | | | |

## Revendications

1. Composé appartenant à la famille des analogues de 15-déoxyspergualine, caractérisé en ce qu'il est choisi parmi l'ensemble constitué par :
(i) les composés de formule : 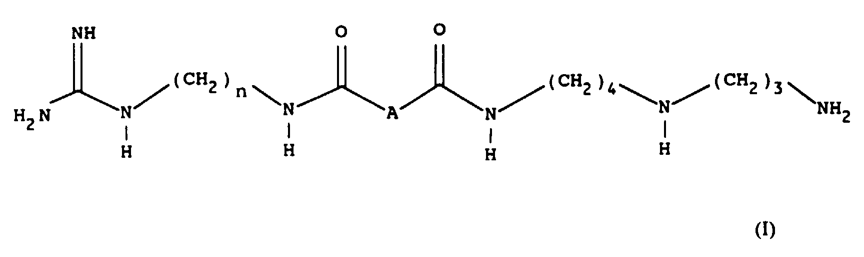 dans laquelle :
- n est égal à 6 ou 8,
- A représente une liaison, un groupe CH₂, un groupe CH(OH), un groupe CHF, un groupe CH(OCH₃), un groupe CH₂NH ou un groupe CH₂O, et,
(ii) leurs sels d'addition.

2. Composé selon la revendication 1, caractérisé en ce que dans la formule I, n est égal à 6.

3. Composé selon la revendication 1, caractérisé en ce que dans la formule I, n est égal à 8.

4. Composé selon la revendication 1, caractérisé en ce que dans la formule I, A représente le groupe CH₂O.

5. Composé selon la revendication 1, caractérisé en ce que dans la formule I, A représente le groupe CH₂.

6. 2-[[[4-[[3-(amino)propyl]amino]butyl]amino]carbonyloxy]-N-[6-[(amino iminométhyl)amino]hexyl]-acétamide, et ses sels d'addition avec un acide minéral ou organique.

7. N-[4-[[3-(amino)propyl]amino]butyl]-N'-[6-[(aminoiminométhyl)amino] hexyl]-propanediamide, et ses sels d'addition avec un acide minéral ou organique.

8. Procédé de préparation d'un composé de formule I ou de l'un de ses sels d'addition selon la revendication I, ledit procédé étant caractérisé en ce qu'il comprend la déprotection d'un composé de formule : 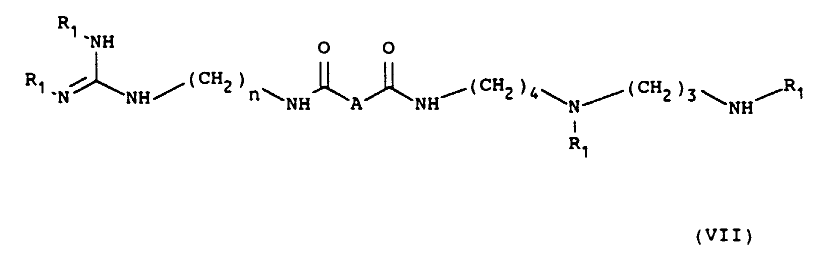 dans laquelle n et A sont définis comme indiqué ci-dessus, et R₁ représente un groupe protecteur de type alcoxycarbonyl, au moyen d'une réaction avec un acide fort, pour le remplacement de R₁ par H.

9. Procédé selon la revendication 8, caractérisé en ce qu'il est choisi parmi l'ensemble constitué par :
- la variante A, qui comprend les étapes consistant à :
(i) faire réagir un composé de formule : 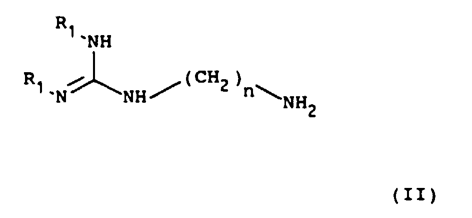 dans laquelle n est égal à 6 ou 8 et R₁ représente un groupe amino-protecteur avec un acide ou un chlorure d'acide de formule : 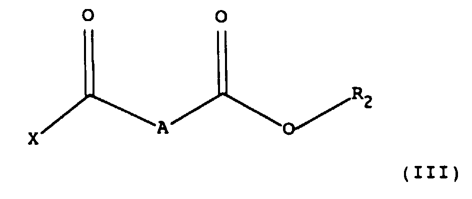 dans laquelle :
- X représente un atome de chlore ou un groupe OH,
- A représente une simple liaison, un groupe CH₂, un groupe CHF, un groupe CH(OCH₂C₆H₅) ou un groupe CH(OCH₃), et,
- R₂ représente un groupe alkyle en C₁-C₃, linéaire ou ramifié, ou un groupe phénylméthyle,
dans un solvant organique, en présence d'un activateur de groupe carboxylique et d'un agent nucléophile, à une température comprise entre 0°C et 40°C, à raison de 1 mole de II pour 1 mole de III, pour obtenir un composé de formule : 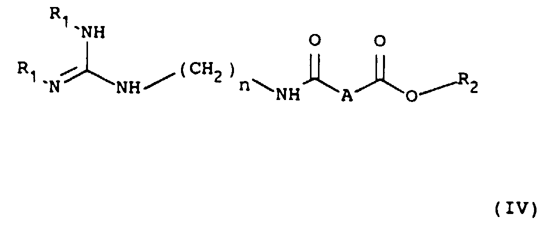 dans laquelle R₁, R₂ et n ont les significations indiquées ci-dessus, et A représente une simple liaison, CH₂, CHF, CH(OCH₂C₆H₅) ou CH(OCH₃),
(ii) saponifier le composé de formule IV, ainsi obtenu, dans un solvant organique en présence d'une base forte pour obtenir un composé de formule : 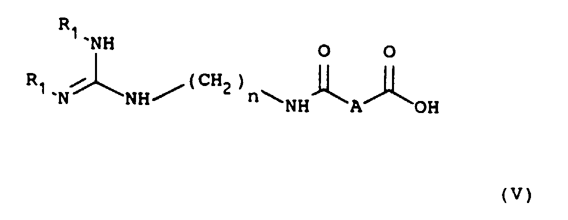 dans laquelle R₁ et n ont les significations indiquées ci-dessus, et A représente une simple liaison, CH₂, CHF, CH(OCH₂C₆H₅) ou CH(OCH₃),
(iii) condenser le composé de formule V, ainsi obtenu, sur une amine de formule : 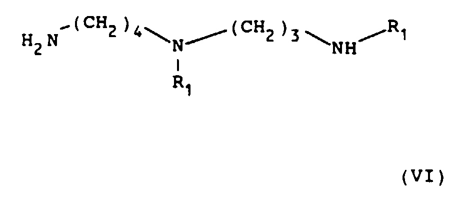 dans laquelle R₁ a la signification indiquée ci-dessus,
dans des conditions identiques à celles de l'étape (i), pour obtenir un composé de formule : 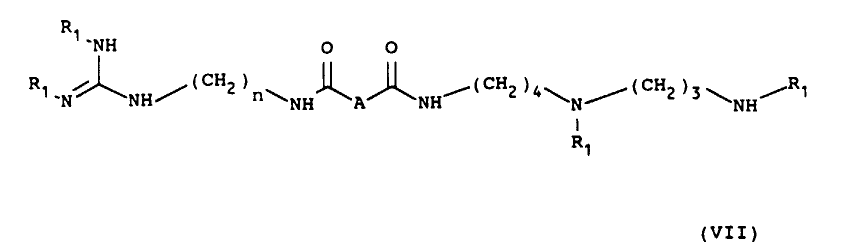
(iv) si nécessaire, déprotéger le composé de formule VII dans laquelle A représente un groupe CH(OCH₂C₆H₅), par hydrogénation catalytique, pour obtenir le composé de formule VII dans laquelle A représente le groupe CH(OH),
(v) déprotéger le composé VII obtenu à l'étape (iii) ou (iv), où A représente une simple liaison, CH₂, CHF, CH(OH) ou CH(OCH₃), pour éliminer le groupe protecteur R₁, par action d'un acide fort et obtenir ainsi un sel d'addition d'un composé de formule I, où A est une simple liaison, CH₂, CHF, CH(OH) ou CH(OCH₃), et
(vi) si nécessaire, obtenir ledit composé de formule I sous forme de base libre par action d'une base forte, puis à partir de ladite base libre, les autres sels d'addition ;
- la variante B qui comprend les étapes consistant à :
(i) faire réagir un composé de formule : 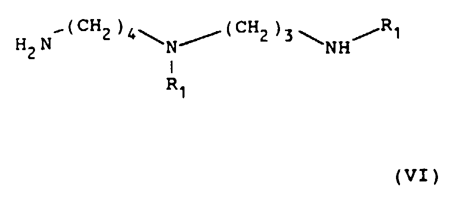 dans laquelle R₁ représente un groupe protecteur comme indiqué ci-dessus, avec un acide ou un chlorure d'acide de formule : 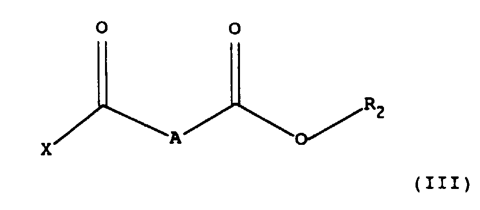 dans laquelle :
- X représente un atome de chlore ou un groupe OH,
- A représente une simple liaison, un groupe CH₂ un groupe CHF, un groupe CH(OCH₂C₆H₅) ou un groupe CH(OCH₃), et,
- R₂ représente un groupe alkyle en C₁-C₃, linéaire ou ramifié, ou un groupe phénylméthyl,
dans un solvant organique, en présence d'un activateur de groupe carboxylique et d'un agent nucléophile, à une température comprise entre 0°C et 40°C, à raison de 1 mole de VI pour 1 mole de III, pour obtenir un composé de formule : 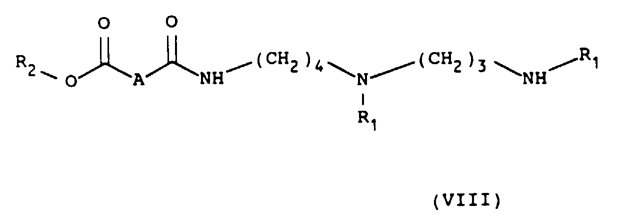 dans laquelle R₁ et R₂ ont les significations indiquées ci-dessus, et A représente une simple liaison, CH₂ CHF, CH(OCH₃), ou CH(OCH₂C₆H₅).
(ii) saponifier le composé de formule VIII, ainsi obtenu, dans un solvant organique en présence d'une base forte pour obtenir un composé de formule : 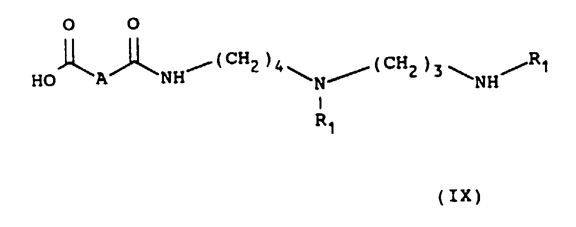 dans laquelle R₁ a les significations indiquées ci-dessus, et A représente une simple liaison, CH₂, CHF, CH(OCH₃), ou CH(OCH₂C₆H₅).
(iii) condenser le composé de formule IX, ainsi obtenu, sur une amine de formule : 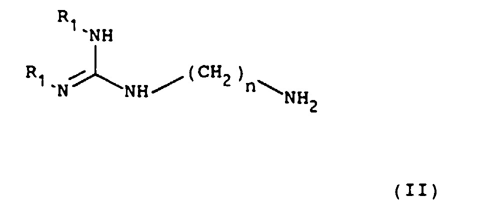 dans laquelle n est égal à 6 ou 8, et R₁ est défini comme indiqué ci-dessus,
dans des conditions indentiques à celles de l'étape (i) ci-dessus,
pour obtenir un composé de formule : 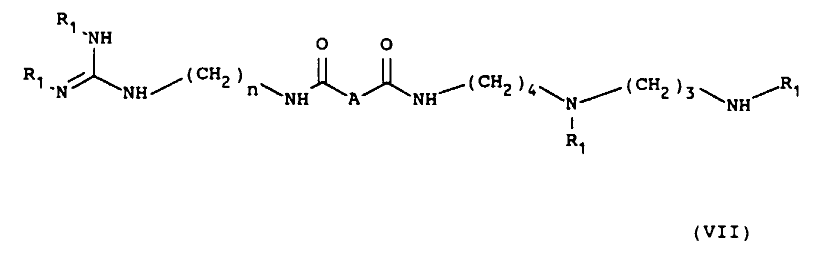 dans laquelle R₁ et n sont définis comme indiqué ci-dessus et A représente une simple liaison, CH₂, CHF, CH(OCH₃), ou CH(OCH₂C₆H₅).
(iv) si nécessaire, déprotéger le composé de formule VII dans laquelle A représente un groupe CH(OCH₂C₆H₅), par hydrogénération catalytique, pour obtenir le composé de formule VII dans laquelle A représente le groupe CH(OH),
(v) déprotéger le composé VII, ainsi obtenu, par action d'un acide fort, pour obtenir un sel d'addition d'un composé de formule I, où A est une simple liaison, CH₂ CHF, CH(OCH₃) ou CH(OH),
(vi) si nécessaire, obtenir ledit composé de formule I sous forme de base libre par action d'une base forte, puis à partir de ladite base libre, les autres sels d'addition ;
- la variante C qui comprend les étapes consistant à :
(i) faire réagir l'extrémité NH₂-terminale d'une base de formule : 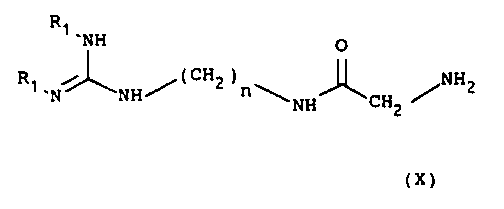 dans laquelle R₁ représente un groupe protecteur comme indiqué ci-dessus, et n est égal à 6 ou 8,
avec un chloroformiate d'alkyle ou un carbonate symétrique, dans un solvant inerte, à la température ambiante (15-25°C),
(ii) aminolyser le composé, ainsi obtenu, par une amine de formule : 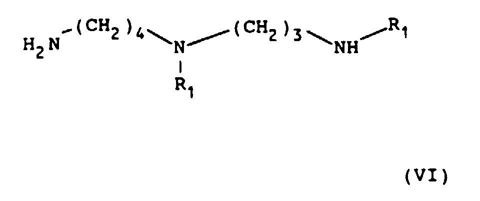 dans laquelle R₁ a la signification indiquée ci-dessus, pour obtenir un composé de formule : 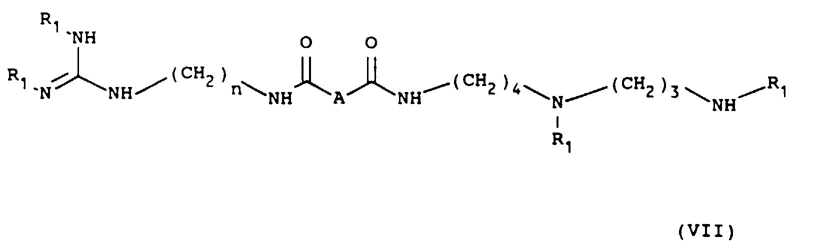 dans laquelle R₁ et n ont les significations indiquées ci-dessus, et A représente le groupe CH₂NH,
(iii) déprotéger le composé VII, ainsi obtenu, par action d'un acide fort pour obtenir un sel d'addition d'un composé de formule I, où A est CH₂NH, et
(iv) si nécessaire, obtenir ledit composé de formule I sous forme de base libre par action d'une base forte, puis à partir de ladite base libre, les autres sels d'addition ; et
- la variante D qui comprend les étapes consistant à :
(i) faire réagir un composé de formule : 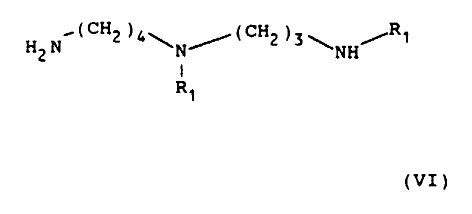 dans laquelle R₁ représente un groupe protecteur comme indiqué ci-dessus, avec un carbonate de formule : 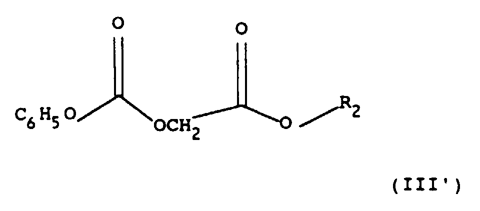 dans laquelle :
- R₂ représente un groupe alkyle en C₁-C₃, linéaire ou ramifié, ou un groupe phénylméthyl,
dans un solvant organique inerte, à la température de reflux du milieu réactionnel, à raison de 1 mole de VI pour 1 mole de III', pour obtenir un composé de formule : 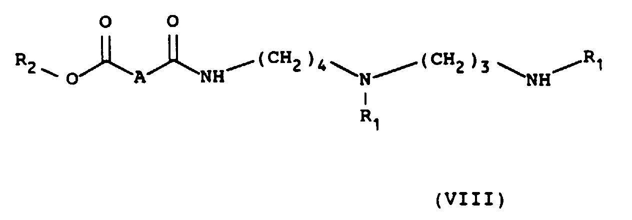 dans laquelle R₁ et R₂ ont les significations indiquées ci-dessus et A représente le groupe CH₂O,
(ii) déprotéger le composé de formule VIII, ainsi obtenu, dans un solvant organique en présence d'une base forte ou par hydrogénation catalytique, pour obtenir un composé de formule : 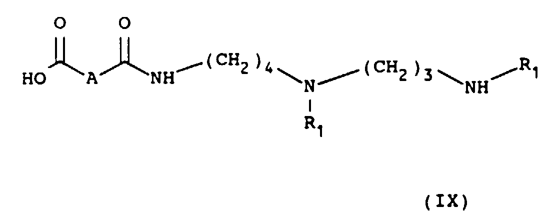 dans laquelle R₁ est défini comme indiqué ci-dessus et A représente CH₂O,
(iii) condenser le composé de formule IX, ainsi obtenu, sur une amine de formule : 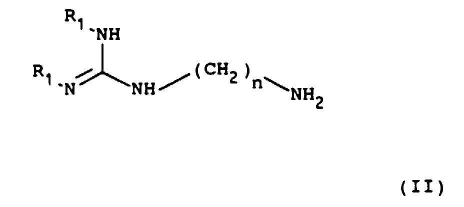 dans laquelle R₁ est défini comme indiqué ci-dessus, et n est égal à 6 ou 8, dans un solvant organique, en présence d'un activateur de groupe carboxylique et en présence d'un agent nucléophile, à une température comprise entre 0°C et 40°C, à raison de 1 mole de IX pour 1 mole de II, pour obtenir un composé de formule : 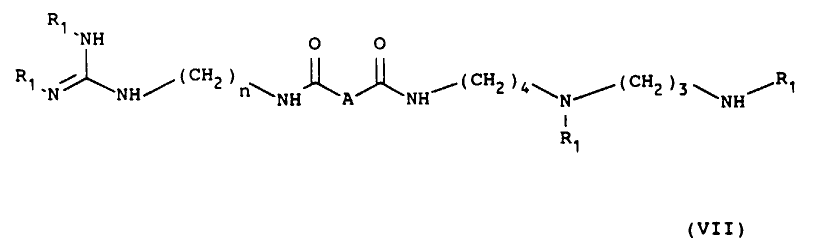 dans laquelle R₁ et n sont définis comme indiqué ci-dessus et A représente CH₂O,
(iv) déprotéger le composé VII, ainsi obtenu, par action d'un acide fort pour obtenir un sel d'addition d'un composé de formule I où A est CH₂O, et
(v) si nécessaire, obtenir ledit composé de formule I sous forme de base libre par action d'une base forte, puis à partir de ladite base libre, les autres sels d'addition.

10. Composition thérapeutique caractérisée en ce qu'elle renferme, en association avec un excipient physiologiquement acceptable, au moins un composé choisi parmi l'ensemble constitué par les composés de formule I et leurs sels d'addition selon la revendication 1.

11. Composé intermédiaire, intervenant dans la synthèse des composés de formule I selon la revendication I caractérisé en ce qu'il est choisi parmi l'ensemble constitué par les composés de formule : 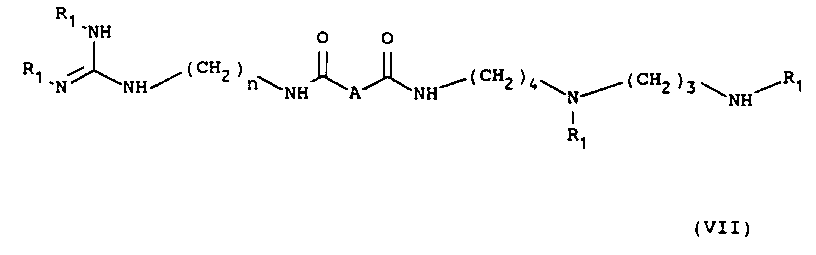 dans laquelle R₁ représente un groupe protecteur, n est égal à 6 ou 8 et A représente une simple liaison, un groupe CH₂, un groupe CH(OH), un groupe CHF, un groupe CH(OCH₃), un groupe CH₂NH, un groupe CH₂O, ou un groupe CH(OCH₂C₆H₅).

12. Utilisation d'une substance immunodépressante choisie parmi l'ensemble constitué par les composés de formule I et leurs sels d'addition non toxiques, selon la revendication 1, pour l'obtention d'un médicament destiné à une utilisation en thérapeutique vis-à-vis des désordres immunitaires.

13. Utilisation d'une substance choisie parmi l'ensemble constitué par les composés de formule I et leurs sels d'addition non toxiques, selon la revendication 1, pour l'obtention d'un médicament destiné à une utilisation en thérapeutique vis-à-vis du paludisme.

## Patentansprüche

1. Verbindung, die zur 15-Desoxyspergualin-Familie gehört, dadurch gekennzeichnet, daß sie aus der Gruppe ausgewählt ist, bestehend aus:
(i) den Verbindungen der Formel: 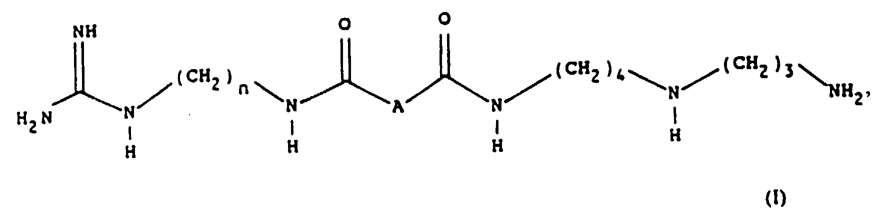 worin:
- n 6 oder 8 ist,
- A eine Bindung, eine CH₂-Guppe, eine CH(OH)-Gruppe, eine CHF-Gruppe, eine CH(OCH₃)-Gruppe, eine CH₂NH-Gruppe oder eine CH₂O-Gruppe darstellt, und (ii) ihren Additionssalzen.

2. Verbindung nach Anspruch 1, dadurch gekennzeichnet, daß n in der Formel 1 gleich 6 ist.

3. Verbindung nach Anspruch 1, dadurch gekennzeichnet, daß n in der Formel 1 gleich 8 ist.

4. Verbindung nach Anspruch 1, dadurch gekennzeichnet, daß A in der Formel 1 die CH₂O-Gruppe darstellt.

5. Verbindung nach Anspruch 1, dadurch gekennzeichnet, daß A in der Formel 1 die CH₂-Gruppe darstellt.

6. 2-[[[4-[[3-(Amino)propyl]amino]butyl]amino]carbonyloxy]-N-[6-[(aminoiminomethyl)amino]hexyl]acetamid und seine Additionssalze mit einer anorganischen oder organischen Säure.

7. N-[4-[[3-(amino)propyl]amino]butyl-N'-[6-[(aminoiminomethyl)amino]hexyl]propandiamid und seine Additionssalze mit einer anorganischen oder organischen Säure.

8. Verfahren zur Herstellung einer Verbindung der Formel 1 oder eines ihrer Additionssalze nach Anspruch 1, wobei das Verfahren dadurch gekennzeichnet ist, daß es umfaßt: Entfernung der Schutzgruppe von einer Verbindung der Formel: 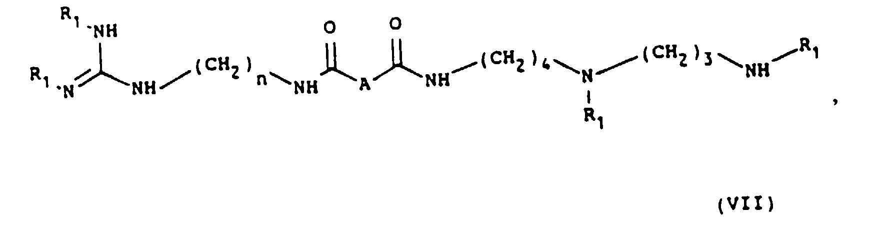 worin n und A wie vorstehend beschrieben definiert sind und R₁ eine Schutzgruppe vom Alkoxycarbonyl-Typ darstellt, durch eine Umsetzung mit einer starken Säure, um R₁ durch H zu ersetzen.

9. Verfahren nach Anspruch 8, dadurch gekennzeichnet, daß es aus der Gruppe ausgewählt ist, bestehend aus:
- der Variante A, die die Schritte umfaßt, bestehend aus:
(i) Reagierenlassen einer Verbindung der Formel: 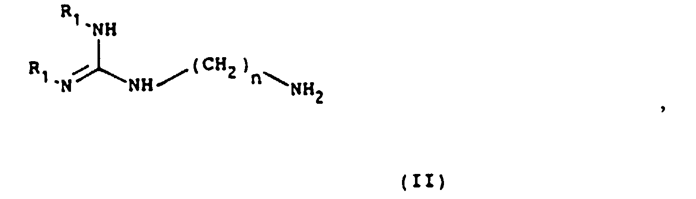 worin n 6 oder 8 ist und R₁ eine Aminoschutzgruppe darstellt, mit einer Säure oder einem Säurechlorid der Formel: 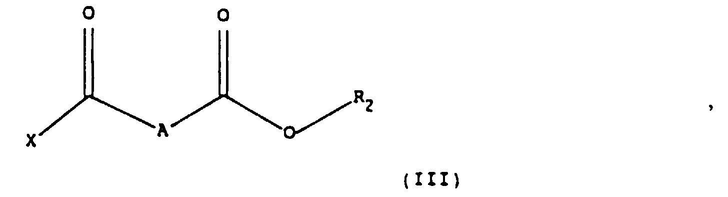 worin:
- X ein Chloratom oder eine OH-Gruppe darstellt,
- A eine Einfachbindung, eine CH₂-Gruppe, eine CHF-Gruppe, eine CH(OCH₂C₆H₅)-Gruppe oder eine CH(OCH₃)-Gruppe darstellt und R₂ eine gerade oder verzweigte C₁-C₃-Alkylgruppe oder eine Phenylmethylgruppe darstellt,
in einem organischen Lösungsmittel in Gegenwart eines Carboxylgruppen-Aktivators und eines Nukleophils, bei einer Temperatur zwischen 0°C und 40°C, bei einem Verhältnis von 1 Mol II zu einem 1 Mol III, um eine Verbindung der Formel: 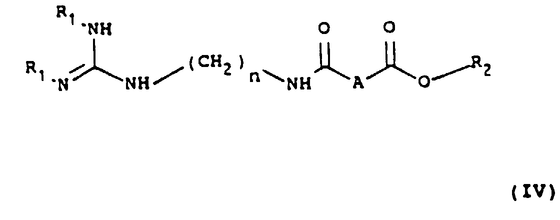 zu erhalten, worin R₁, R₂ und n die vorstehend beschriebenen Bedeutungen haben und A eine Einfachbindung, CH₂, CHF, CH(OCH₂C₆H₅) oder CH(OCH₃) darstellt,
(ii) Verseifen der so erhaltenen Verbindung der Formel IV in einem organischen Lösungsmittel in Gegenwart einer starken Base, um eine Verbindung der Formel: 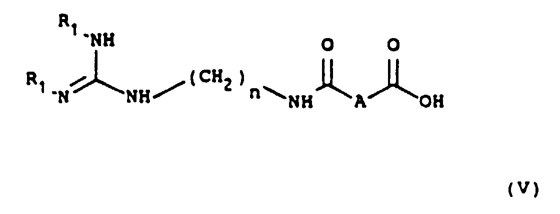 zu erhalten, worin R₁ und n die vorstehend beschriebenen Bedeutungen haben und A eine Einfachbindung, CH₂, CHF, CH(OCH₂C₆H₅) oder CH(OCH₃) darstellt,
(iii) Kondensieren der so erhaltenen Verbindung der Formel V mit einem Amin der Formel: 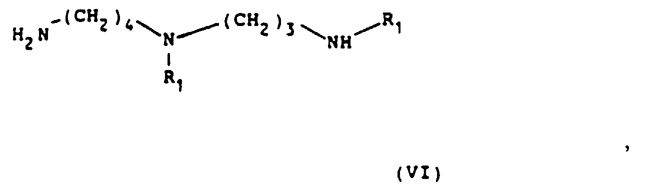 worin R₁ die vorstehend beschriebene Bedeutung hat, unter gleichen Bedingungen wie bei Schritt (i), um eine Verbindung der Formel: 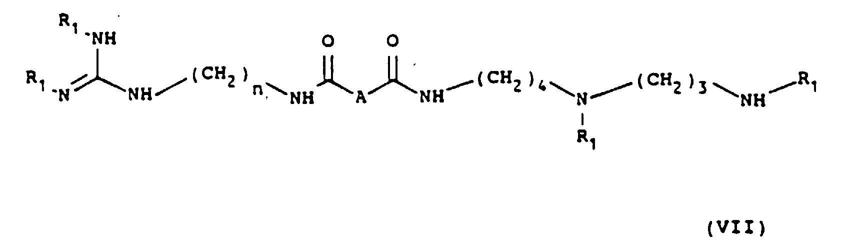 zu erhalten,
(iv) falls nötig, Entfernen der Schutzgruppe von der Verbindung der Formel VII, worin A eine CH(OCH₂C₆H₅)-Gruppe darstellt, durch katalytische Hydrierung, um eine Verbindung der Formel VII zu erhalten, worin A eine CH(OH)-Gruppe darstellt,
(v) Entfernen der Schutzgruppe von der in Schritt (iii) oder (iv) erhaltenen Verbindung der Formel VII, wobei A eine Einfachbindung, CH₂, CHF, CH(OH) oder CH(OCH₃) darstellt, um die Schutzgruppe R₁ zu eliminieren, durch die Wirkung einer starken Säure und Erhalten eines Additionssalz einer Verbindung der Formel I, wobei A eine Einfachbindung, CH₂, CHF, CH(OH) oder CH(OCH₃) ist, und
(vi) falls nötig, Erhalten der vorstehenden Verbindung der Formel I in Form der freien Base durch die Wirkung einer starken Base, dann, ausgehend von der vorstehenden freien Base, der anderen Additionssalze;
- der Variante B, die die Schritte umfaßt, bestehend aus:
(i) Reagierenlassen einer Verbindung der Formel: 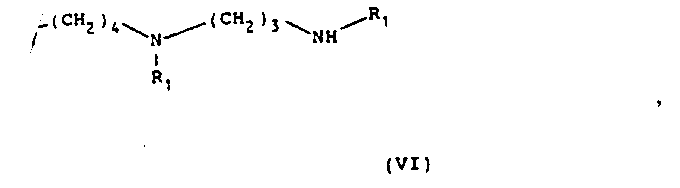 worin R₁ eine Schutzgruppe wie vorstehend beschrieben darstellt, mit einer Säure oder einem Säurechlorid der Formel: 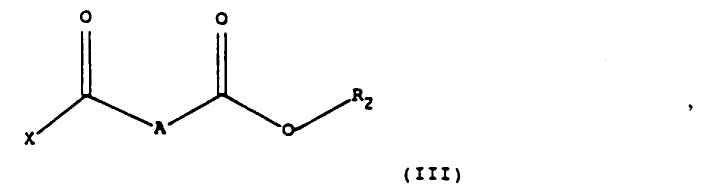 worin:
- X ein Chloratom oder eine OH-Gruppe darstellt,
- A eine Einfachbindung, eine CH₂-Gruppe, eine CHF-Gruppe, eine CH(OCH₂C₆H₅)-Gruppe oder eine CH(OCH₃)-Gruppe darstellt und R₂ eine gerade oder verzweigte C₁-C₃-Alkylgruppe oder eine Phenylmethylgruppe darstellt,
in einem organischen Lösungsmittel in Gegenwart eines Carboxylgruppen-Aktivators und eines Nukleophils, bei einer Temperatur zwischen 0°C und 40°C, bei einem Verhältnis von 1 Mol VI zu einem 1 Mol III, um eine Verbindung der Formel: 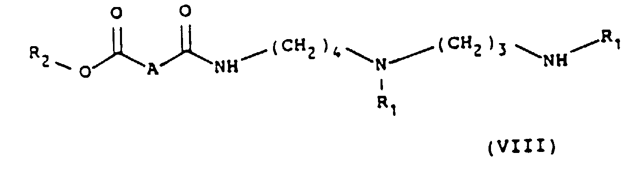 zu erhalten, worin R₁ und R₂ die vorstehend beschriebenen Bedeutungen haben und A eine Einfachbindung, CH₂, CHF, CH(OCH₃) oder CH(OCH₂C₆H₅) darstellt,
(ii) Verseifen der so erhaltenen Verbindung der Formel VIII in einem organischen Lösungsmittel in Gegenwart einer starken Base, um eine Verbindung; der Formel: 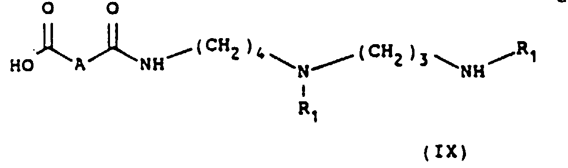 zu erhalten, worin R₁ die vorstehend beschriebenen Bedeutungen hat und A eine Einfachbindung, CH₂, CHF, CH(OCH₃) oder CH(OCH₂C₆H₅) darstellt,
(iii) Kondensieren der so erhaltenen Verbindung der Formel IX mit einem Amin der Formel: 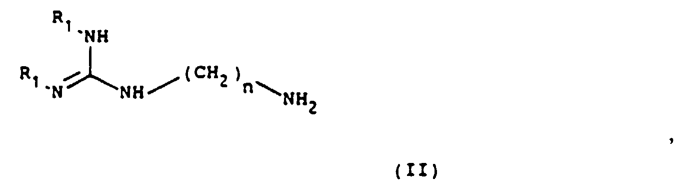 worin n 6 oder 8 ist und R₁ wie vorstehend beschrieben definiert ist, unter gleichen Bedingungen, wie die des vorstehenden Schrittes (i), um eine Verbindung der Formel: 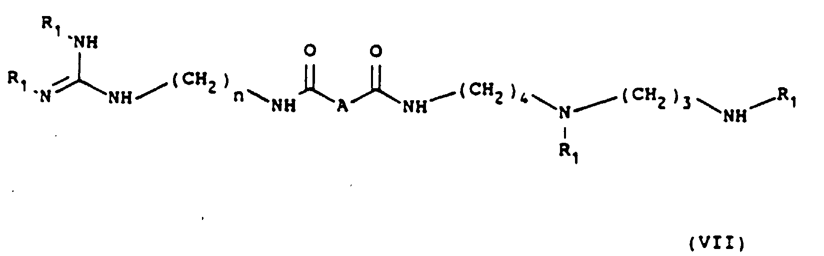 zu erhalten, worin R₁ und n wie vorstehend beschrieben definiert sind und A eine Einfachbindung, CH₂, CHF, CH(OCH₃) oder CH(OCH₂C₆H₅) darstellt,
(iv) falls nötig, Entfernen der Schutzgruppe von der Verbindung der Formel VII, worin A eine CH(OCH₂C₆H₅)-Gruppe darstellt, durch katalytische Hydrierung, um eine Verbindung der Formel VII zu erhalten, worin A eine CH(OH)-Gruppe darstellt,
(v) Entfernen der Schutzgruppe von der so erhaltenen Verbindung VII durch die Wirkung einer starken Säure, um ein Additionssalz einer Verbindung der Formel I zu erhalten, wobei A eine Einfachbindung, CH₂, CHF, CH(OCH₃) oder CH(OH) ist,
(vi) falls nötig, Erhalten der vorstehenden Verbindung der Formel I in Form der freien Base durch die Wirkung einer starken Base, dann, ausgehend von der vorstehenden freien Base, der anderen Additionssalze;
- der Variante C, die die Schritte umfaßt, bestehend aus:
(i) Reagierenlassen des NH₂-Terminus einer Base der Formel: 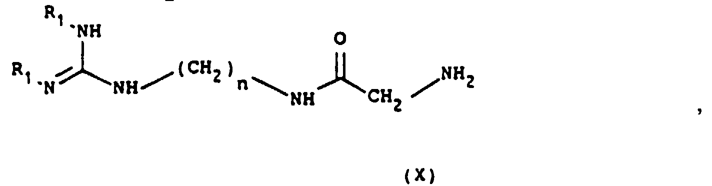 worin R₁ eine Schutzgruppe wie vorstehend beschrieben darstellt und n 6 oder 8 ist, mit einem Alkylchlorformiat oder einem symmetrischen Carbonat in einem inerten Lösungsmittel bei Umgebungstemperatur (15-25°C).
(ii) Aminolysieren der so erhaltenen Verbindung durch ein Amin der Formel: 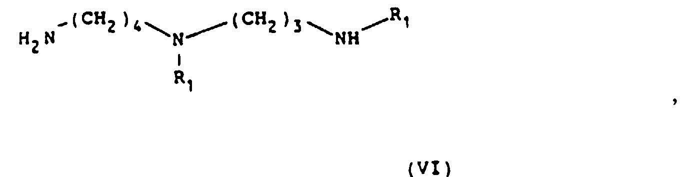 worin R₁ die vorstehend beschriebene Bedeutung hat, um eine Verbindung der Formel: 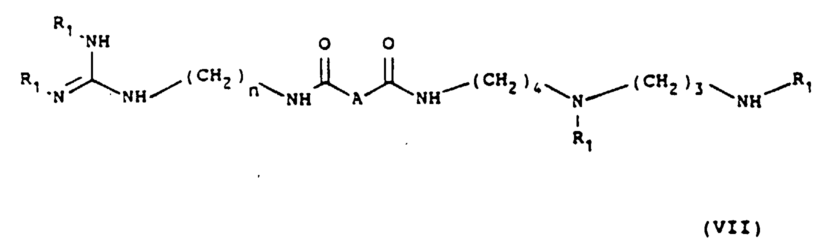 zu erhalten, worin R₁ und n die vorstehend beschriebenen Bedeutungen haben und A eine CH₂NH-Gruppe darstellt,
(iii) Entfernen der Schutzgruppe von der so erhaltenen Verbindung VII durch die Wirkung einer starken Säure, um ein Additionssalz einer Verbindung der Formel I zu erhalten, wobei A CH₂NH ist, und,
(iv) falls nötig, Erhalten der vorstehenden Verbindung der Formel I in Form der freien Base durch die Wirkung einer starken Base, dann, ausgehend von der vorstehenden freien Base, der anderen Additionssalze; und
- der Variante D, die die Schritte umfaßt, bestehend aus:
(i) Reagierenlassen einer Verbindung der Formel: 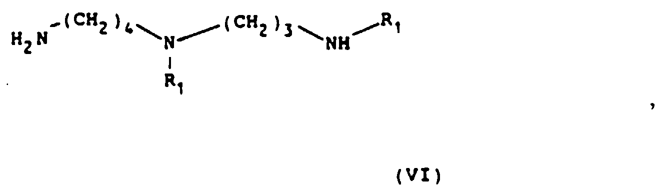 worin R₁ eine Schutzgruppe wie vorstehend beschrieben darstellt, mit einem Carbonat der Formel: 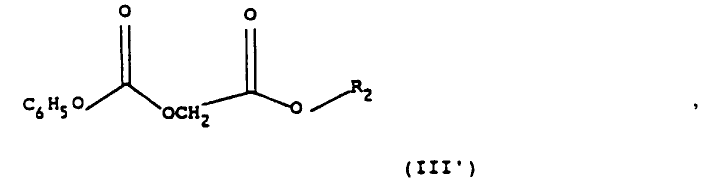 worin:
- R₂ eine gerade oder verzweigte C₁-C₃-Alkylgruppe oder eine Phenylmethylgruppe darstellt,
in einem inerten organischen Lösungsmittel bei der Rückflußtemperatur des Reaktionsmediums bei einem Verhältnis von 1 Mol VI zu einem 1 Mol III', um eine Verbindung der Formel: 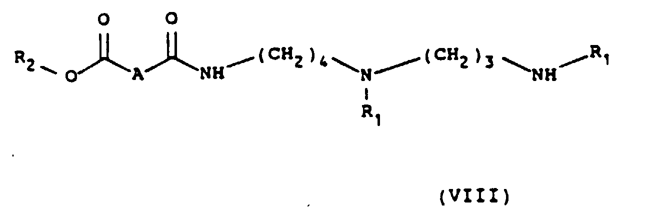 zu erhalten, worin R₁ und R₂ die vorstehend beschriebenen Bedeutungen haben und A eine CH₂O-Gruppe darstellt,
(ii) Entfernen der Schutzgruppe von der so erhaltenen Verbindung der Formel VIII in einem organischen Lösungsmittel in Gegenwart einer starken Base oder durch katalytische Hydrierung, um eine Verbindung der Formel: 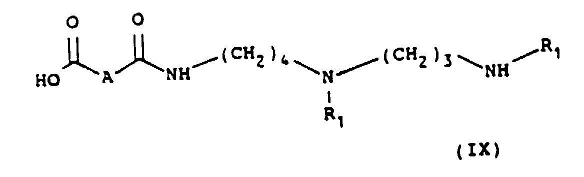 zu erhalten, worin R₁ wie vorstehend beschrieben definiert ist und A CH₂O darstellt,
(iii) Kondensieren der so erhaltenen Verbindung der Formel IX mit einem Amin der Formel: 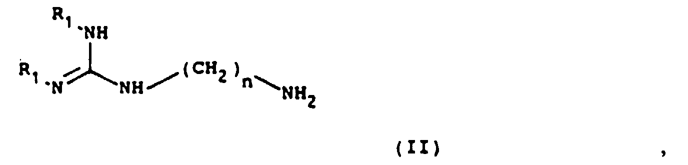 worin R₁ wie vorstehend beschrieben definiert ist und n 6 oder 8 ist, in einem organischen Lösungsmittel in Gegenwart eines Carboxylgruppen-Aktivators und in Gegenwart eines Nukleophils, bei einer Temperatur zwischen 0°C und 40°C, bei einem Verhältnis von 1 Mol IX zu einem 1 Mol II, um eine Verbindung der Formel: 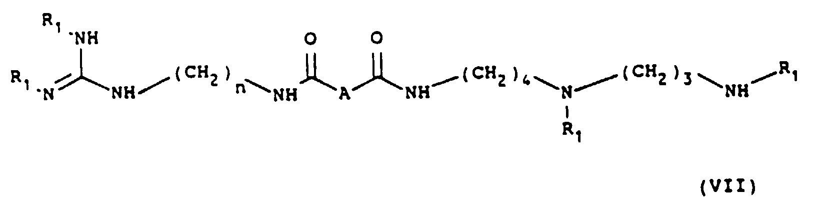 zu erhalten, worin R₁ und n wie vorstehend beschrieben definiert sind und A CH₂O darstellt,
(iv) Entfernen der Schutzgruppe von der so erhaltenen Verbindung VII durch die Wirkung einer starken Säure, um ein Additionssalz einer Verbindung der Formel I zu erhalten, wobei A CH₂O ist, und
(v) falls nötig, Erhalten der vorstehenden Verbindung der Formel I in Form der freien Base durch die Wirkung einer starken Base, dann, ausgehend von der vorstehenden freien Base, der anderen Additionssalze.

10. Therapeutische Zusammensetzung, dadurch gekennzeichnet, daß sie, in Verbindung mit einem physiologisch verträglichen Excipienten, mindestens eine Verbindung enthält, ausgewählt aus der Gruppe, bestehend aus den Verbindungen der Formel I und ihren Additionssalzen nach Anspruch 1.

11. Zwischenverbindung, die bei der Synthese der Verbindungen der Formel I nach Anspruch 1 als Zwischenprodukt auftritt, dadurch gekennzeichnet, daß sie ausgewählt ist aus der Gruppe, bestehend aus den Verbindungen der Formel: 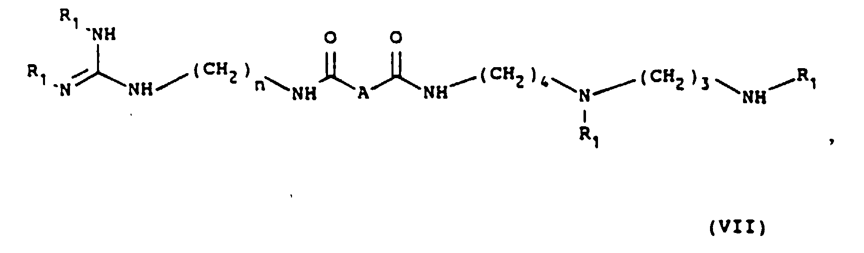 worin R₁ eine Schutzgruppe darstellt, n 6 oder 8 ist und A eine Einfachbindung, eine CH₂-Gruppe, eine CH(OH)-Gruppe, eine CHF-Gruppe, eine CH(OCH₃)-Gruppe, eine CH₂NH-Gruppe, eine CH₂O-Gruppe oder eine CH(OCH₂C₆H₅)-Gruppe darstellt.

12. Verwendung einer immundepressiven Substanz, ausgewählt aus der Gruppe, bestehend aus den nichttoxischen Verbindungen der Formel I und ihren Additionssalzen nach Anspruch 1, um ein für die Verwendung zur Therapie von Immunstörungen bestimmtes Medikament zu erhalten.

13. Verwendung einer Substanz, ausgewählt aus der Gruppe, bestehend aus den nichttoxischen Verbindungen der Formel I und ihren Additionssalzen nach Anspruch 1, um ein für die Verwendung zur Therapie von Malaria bestimmtes Medikament zu erhalten.

## Claims

1. A compound belonging to the family of 15-deoxy-spergualin analogs, which is selected from the group consisting of:
(i) the compounds of the formula 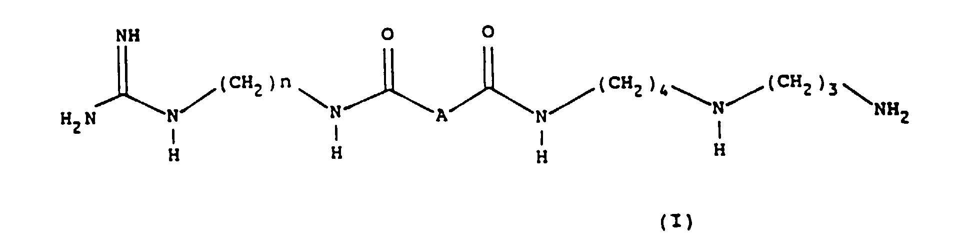 in which:
- n is equal to 6 or 8 and
- A is a bond, a group CH₂, a group CH(OH), a group CHF, a group CH(OCH₃), a group CH₂NH or a group CH₂O, and
(ii) their addition salts.

2. A compound according to claim 1 wherein n is equal to 6 in formula I.

3. A compound according to claim 1 wherein n is equal to 8 in formula I.

4. A compound according to claim 1 wherein A is the group CH₂O in formula I.

5. A compound according to claim 1 wherein A is the group CH₂ in formula I.

6. 2-[[[4-[[3-(Amino)propyl]amino]butyl]amino]carbonyloxy]-N-[6-[(aminoiminomethyl)amino]hexyl]acetamide and its addition salts with a mineral or organic acid.

7. N-[4-[[3-(Amino)propyl]amino]butyl]-N'-[6-[(aminoiminomethyl)amino]hexyl]propanediamide and its addition salts with a mineral or organic acid.

8. A method of preparing a compound of formula I or one of its addition salts according to claim 1, said method comprising the deprotection of a compound of the formula 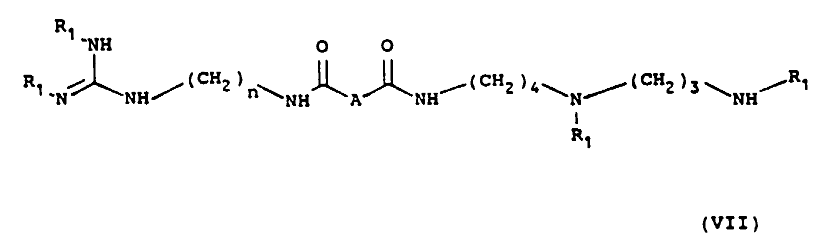 in which n and A are defined as indicated above and R₁ is a protecting group of the alkoxycarbonyl type, by reaction with a strong acid in order to replace R₁ with H.

9. A method according to claim 8 which is selected from the group consisting of:
- variant A, which comprises steps consisting in:
(i) reacting a compound of the formula 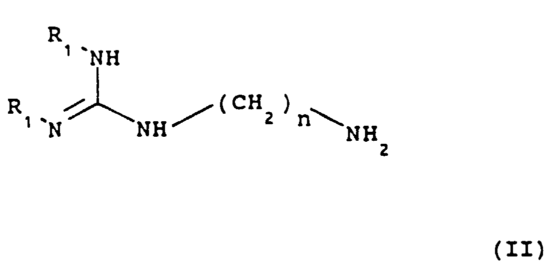 in which n is equal to 6 or 8 and R₁ is an amino-protecting group, with an acid or an acid chloride of the formula 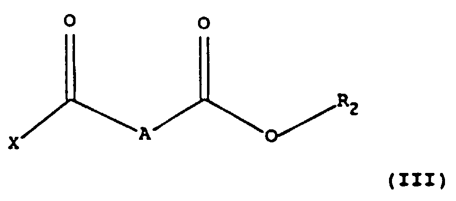 in which:
- X is a chlorine atom or a group OH,
- A is a single bond, a group CH₂, a group CHF, a group CH(OCH₃C₆H₅) or a group CH(OCH₃) and
- R₂ is a linear or branched C₁-C₃-alkyl group or a phenylmethyl group,
in an organic solvent, in the presence of a carboxy group activator and a nucleophilic agent, at a temperature between 0°C and 40°C, at a rate of 1 mol of II to 1 mol of III, to give a compound of the formula 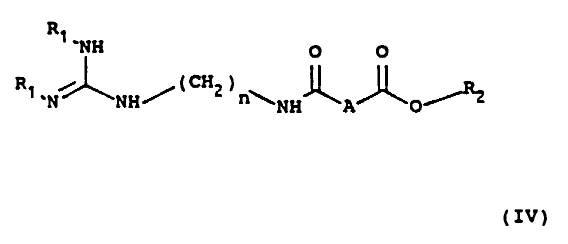 in which R₁, R₂ and n are defined as indicated above and A is a single bond, CH₂, CHF, CH(OCH₂C₆H₅) or CH(OCH₃),
(ii) saponifying the resulting compound of formula IV in an organic solvent, in the presence of a strong base, to give a compound of the formula 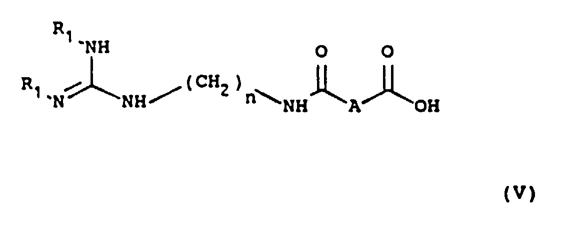 in which R₁ and n are defined as indicated above and A is a single bond, CH₂, CHF, CH(OCH₂C₆H₅) or CH(OCH3),
(iii) condensing the resulting compound of formula V with an amine of the formula 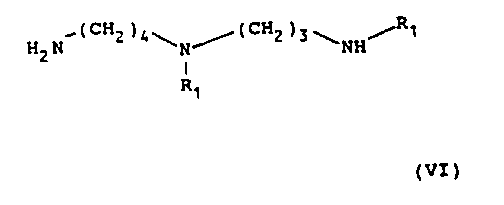 in which R₁ is defined as indicated above, under conditions identical to those of step (i), to give a compound of the formula 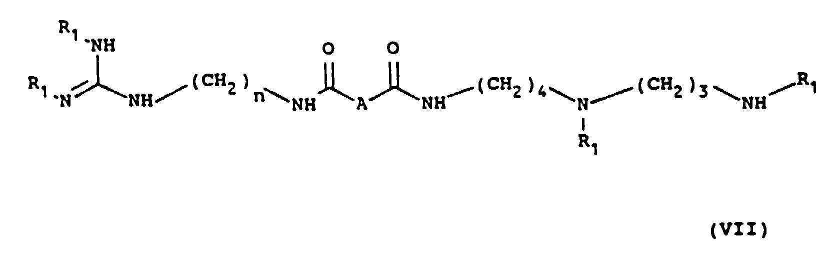
(iv) if necessary, deprotecting the compound of formula VII in which A is a group CH(OCH₂C₆H₅) by catalytic hydrogenation to give the compound of formula VII in which A is the group CH(OH),
(v) deprotecting the compound VII obtained in step (iii) or (iv) where A is a single bond, CH₂, CHF, CH(OH) or CH(OCH₃) to remove the protecting group R₁, by reaction with a strong acid, thereby giving an addition salt of a compound of formula I where A is a single bond, CH₂, CHF, CH(OH) or CH(OCH₃), and
(vi) if necessary, obtaining said compound of formula I in the form of the free base by reaction with a strong base, and then obtaining the other addition salts from said free base;
- variant B, which comprises steps consisting in:
(i) reacting a compound of the formula 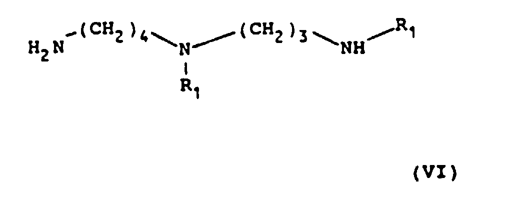 in which R₁ is a protecting group as indicated above, with an acid or an acid chloride of the formula 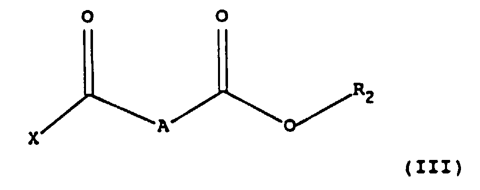 in which:
- X is a chlorine atom or a group OH,
- A is a single bond, a group CH₂, a group CHF, a group CH(OCH₂C₆H₅) or a group CH(OCH₃) and
- R₂ is a linear or branched C₁-C₃-alkyl group or a phenylmethyl group,
in an organic solvent, in the presence of a carboxy group activator and a nucleophilic agent, at a temperature between 0°C and 40°C, at a rate of 1 mol of VI to 1 mol of III, to give a compound of the formula 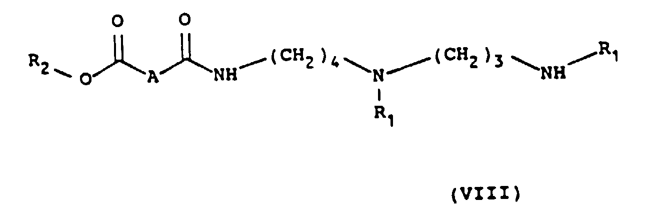 in which R₁ and R₂ are defined as indicated above and A is a single bond, CH₂, CHF, CH(OCH₃) or CH(OCH₂C₆H₅),
(ii) saponifying the resulting compound of formula VIII in an organic solvent, in the presence of a strong base, to give a compound of the formula 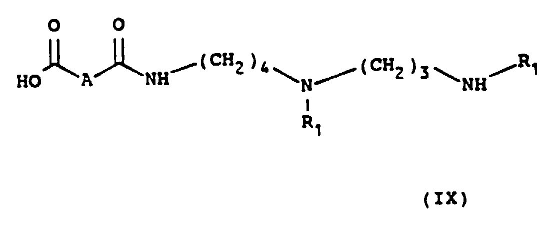 in which R₁ is defined as indicated above and A is a single bond, CH₂, CHF, CH(OCH₃) or CH(OCH₂C₆H₅),
(iii) condensing the resulting compound of formula IX with an amine of the formula 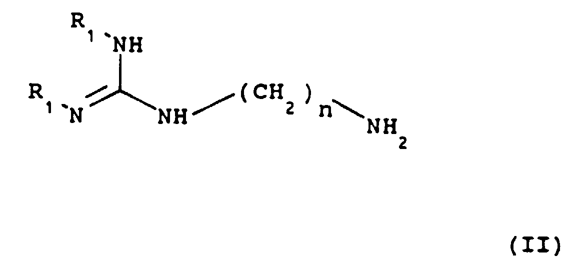 in which n is equal to 6 or 8 and R₁ is defined as indicated above, under conditions identical to those of step (i) above, to give a compound of the formula 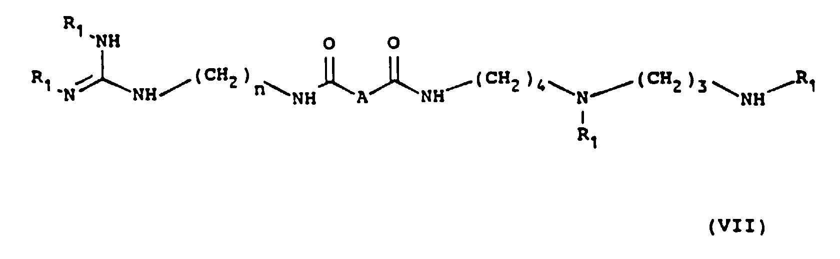 in which R₁ and n are defined as indicated above and A is a single bond, CH₂, CHF, CH(OCH₃) or CH(OCH₂C₆H₅), (iv) if necessary, deprotecting the compound of formula VII in which A is a group CH(OCH₂C₆H₅) by catalytic hydrogenation to give the compound of formula VII in which A is the group CH(OH),
(v) deprotecting the resulting compound VII by reaction with a strong acid to give an addition salt of a compound of formula I where A is a single bond, CH₂, CHF, CH(OCH₃) or CH(OH), and
(vi) if necessary, obtaining said compound of formula I in the form of the free base by reaction with a strong base, and then obtaining the other addition salts from said free base;
- variant C, which comprises steps consisting in:
(i) reacting the NH₂-terminal end of a base of the formula 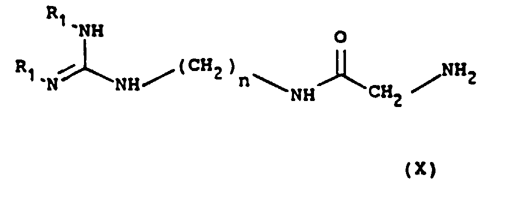 in which R₁ is a protecting group as indicated above and n is equal to 6 or 8, with an alkyl chloroformate or a symmetrical carbonate, in an inert solvent, at room temperature (15-25°C),
(ii) aminolyzing the resulting compound with an amine of the formula 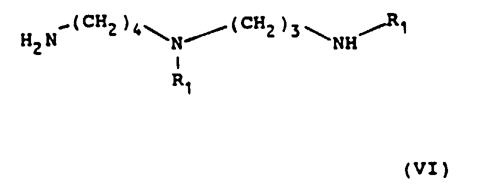 in which R₁ is defined as indicated above, to give a compound of the formula 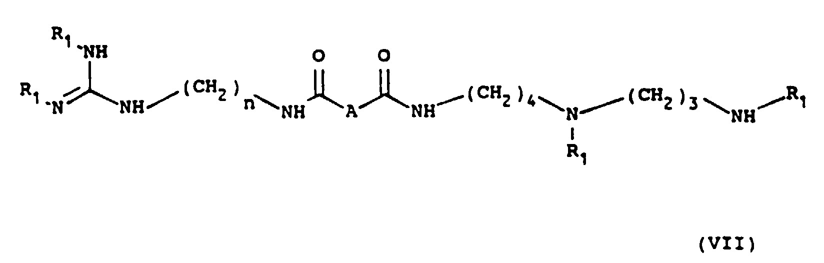 in which R₁ and n are defined as indicated above and A is the group CH₂NH,
(iii) deprotecting the resulting compound VII by reaction with a strong acid to give an addition salt of a compound of formula I where A is CH₂NH, and
(iv) if necessary, obtaining said compound of formula I in the form of the free base by reaction with a strong base, and then obtaining the other addition salts from said free base; and
- variant D, which comprises steps consisting in:
(i) reacting a compound of the formula 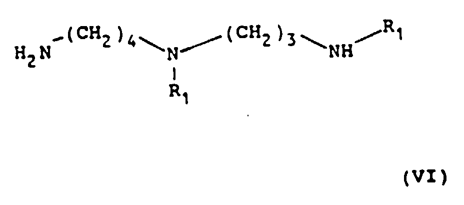 in which R₁ is a protecting group as indicated above, with a carbonate of the formula 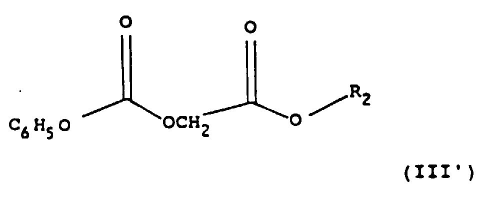 in which:
- R₂ is a linear or branched C₁-C₃-alkyl group or a phenylmethyl group,
in an inert organic solvent, at the reflux temperature of the reaction medium, at a rate of 1 mol of VI to 1 mol of III', to give a compound of the formula 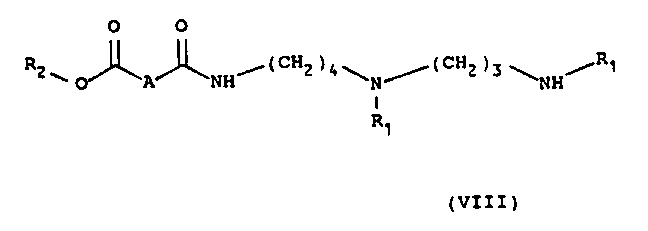 in which R₁ and R₂ are defined as indicated above and A is the group CH₂O,
(ii) deprotecting the resulting compound of formula VIII in an organic solvent, in the presence of a strong base or by catalytic hydrogenation, to give a compound of the formula 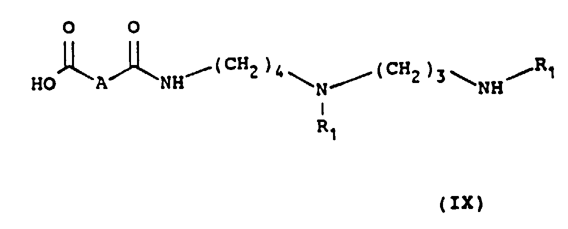 in which R₁ is defined as indicated above and A is CH₂O,
(iii) condensing the resulting compound of formula IX with an amine of the formula 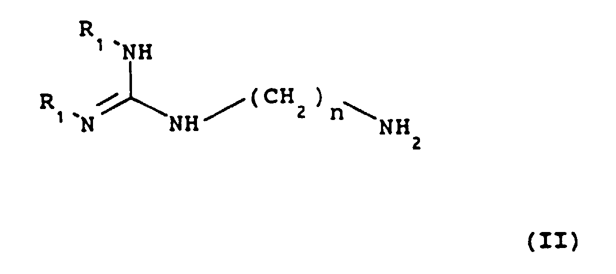 in which R₁ is defined as indicated above and n is equal to 6 or 8, in an organic solvent, in the presence of a carboxy group activator and in the presence of a nucleophilic agent, at a temperature between 0°C and 40°C, at a rate of 1 mol of IX to 1 mol of II, to give a compound of the formula 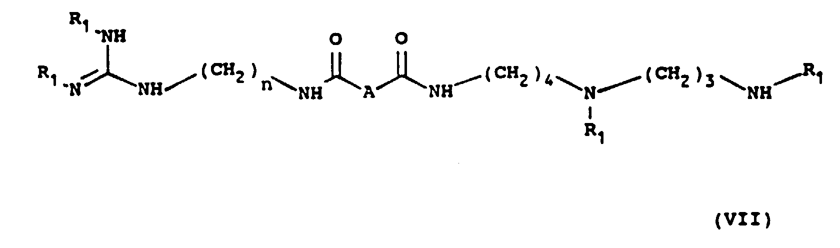 in which R₁ and n are defined as indicated above and A is CH₂O,
(iv) deprotecting the resulting compound VII by reaction with a strong acid to give an addition salt of a compound of formula I where A is CH₂O, and
(v) if necessary, obtaining said compound of formula I in the form of the free base by reaction with a strong base, and then obtaining the other addition salts from said free base.

10. A therapeutic composition which contains, in association with a physiologically acceptable excipient, at least one compound selected from the group consisting of the compounds of formula I and their addition salts according to claim 1.

11. An intermediate involved in the synthesis of the compounds of formula I according to claim 1, which is selected from the group consisting of the compounds of the formula 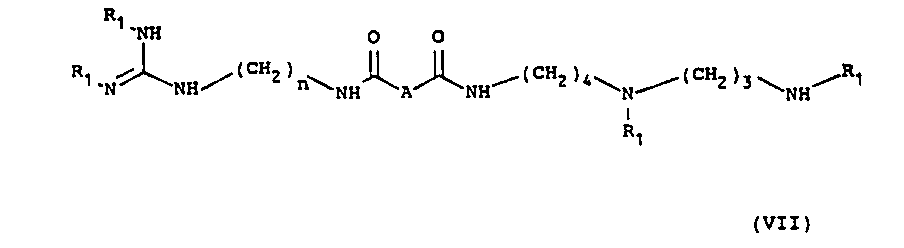 in which R₁ is a protecting group, n is equal to 6 or 8 and A is a single bond, a group CH₂, a group CH(OH), a group CHF, a group CH(OCH₃), a group CH₂NH, a group CH₂O or a group CH(OCH₂C₆H₅).

12. Use of an immunosuppressive substance selected from the group consisting of the compounds of formula I and their non-toxic addition salts, according to claim 1, for the preparation of a drug intended for use in therapeutics to combat immune disorders.

13. Use of a substance selected from the group consisting of the compounds of formula I and their non-toxic addition salts, according to claim 1, for the preparation of a drug intended for use in therapeutics to combat malaria.
